# EUROPEAN PATENT SPECIFICATION

(11) **EP 2 509 997 B1**
(45) Date of publication and mention of the grant of the patent: **30.08.2017**
(21) Application number: 10796219.3
(22) Date of filing: 07.12.2010
(51) Int. Cl.: C07K 16/00, C07K 14/605, C07K 14/575, A61K 38/22, A61K 38/26

(54) **CONJUGATES COMPRISING AN ANTIBODY SURROGATE SCAFFOLD WITH IMPROVED PHARMACOKINETIC PROPERTIES**
KONJUGATE MIT EINEM ANTKÖRPERSURROGATGERÜST MIT VERBESSERTEN PHARMAKOKINETISCHEN EIGENSCHAFTEN
CONJUGUÉS COMPRENANT UN ÉCHAFAUDAGE DE SUBSTITUTS D'ANTICORPS PRÉSENTANT DES PROPRIÉTÉS PHARMACOCINÉTIQUES AMÉLIORÉES

(30) Priority: 07.12.2009 US 267408 P
(43) Date of publication of application: 17.10.2012
(73) Proprietor: i2 Pharmaceuticals, Inc., Boulder, CO 8030 (US)
(72) Inventor: HOROWITZ, Lawrence, Atherton, CA 94027 (US); BHATT, Ramesh, R., Belmont, CA 94002 (US); KURTZMAN, Aaron, L., San Carlos, CA 94070 (US)
(74) Representative: Denison, Christopher Marcus
(86) International application number: PCT/US2010/059342
(87) International publication number: WO 2011/071957

(56) References cited:
- WO-A1-2007/040437
- WO-A2-2006/059106
- WO-A2-2008/036449
- WO-A2-2008/118970
- WO-A2-2010/006286
- GREEN B D ET AL: "Lys9 for Glu9 substitution in glucagon-like peptide-1(7-36)amide confers dipeptidylpeptidase IV resistance with cellular and metabolic actions similar to those of established antagonists glucagon-like peptide-1(9-36)amide and exendin (9-39)", METABOLISM, CLINICAL AND EXPERIMENTAL, W.B. SAUNDERS CO., PHILADELPHIA, PA, US, vol. 53, no. 2, 1 February 2004 (2004-02-01), pages 252-259, XP002398090, ISSN: 0026-0495
- XU LI ET AL: "Combinatorial surrobody libraries", PROCEEDINGS OF THE NATIONAL ACADEMY OF SCIENCES OF THE UNITED STATES (PNAS), NATIONAL ACADEMY OF SCIENCE, US, vol. 105, no. 31, 5 August 2008 (2008-08-05), pages 10756-10761, XP002498064, ISSN: 0027-8424, DOI: DOI:10.1073/PNAS.0805293105
- KAGANMAN I: "A surrogate scaffold tested", NATURE METHODS, NATURE PUBLISHING GROUP, GB, vol. 5, no. 10, 1 January 2008 (2008-01-01), page 861, XP002597770, ISSN: 1548-7091, DOI: DOI:10.1038/NMETH1008-861

## Description

### Field of the Invention

The present invention concerns methods and means for modulating pharmacokinetic properties of molecules, such as biologically active molecules. More specifically, the present invention concerns conjugates comprising a biologically active moiety and a moiety conjugated to and modulating at least one pharmacokinetic property of the biologically active moiety (pharmacokinetic property modulating moiety). In particular, the invention concerns conjugates comprising a scaffold including one or more functionally null binding regions conjugated with a biologically active (e.g. drug) moiety the pharmacokinetic properties, such as *in vivo* half-life, of which are to be modulated.

### Background of the Invention

Many peptides and polypeptides have inherently short half-lives, which can prevent the development and clinical use of many otherwise promising drug candidates. Thus, for example, rapid clearance can make the maintenance of therapeutic levels of a drug unfeasible because of cost, amount or frequency of the required dosing.

The long serum half-life of serum albumin has been exploited to develop albumin-conjugated protein drugs that have longer half-lives as compared to the unconjugated protein. This concept has been used to extend the half-lives of a number of proteins including interferon-α, interleukin-2, and G-CSF (Sung C, et al. J Interferon Cytokine Res. 2003 23(1):25-36; and Halpern W, et al., Pharm Res. 2002 19(11):1720-9).

In addition, plasma-protein binding has been described as a means of improving the pharmacokinetic properties of short lived molecules. Serum albumin binding peptides have been described to alter the pharmacodynamics of fused proteins, including alteration of tissue uptake, penetration, and diffusion. These pharmacodynamic parameters were modulated by specific selection of the appropriate serum albumin binding peptide sequence (US 20040001827). For further details see, Dennis et al. J Biol Chem. 2002 277:35035-35043 and WO 01/45746. Protein and peptide conjugates able to bond albumin and having extended half-lives are disclosed in U.S. Patent No. 6,267,964.

In addition, the serum half-lives of antibodies have been extended by incorporating a salvage receptor binding epitope into the antibody, or an antibody fragment, as described, for example, in U.S. Pat. No. 5,739,277.

GLP-1 receptor mimetibody agonists with longer half-lives have been reported (O'Neil, et al. U.S. Patent Neo. 7,833,531).

Fusion proteins constructed from a protein receptor sequence linked to an appropriate immunoglobulin constant domain sequence (immunoadhesins) with extended half-lives are also known in the art. Immunoadhesins are disclosed, for example, in U.S. Patent Nos. 5,116,964; 5,428,130; 5,455,165; 5,514,582; 5,565,335; 5,714,147; 6,406,697, and 6,710,169, and in PCT Publication No. WO 91/08298. In a typical immunoadhesin the receptor sequence is fused C-terminally to the N-terminus of the immunoglobulin constant domain sequence, such as the Fc portion of an immunoglobulin constant domain.

While intact immunoglobulins typically have long serum half-lives on the order of weeks (about 21 days for IgG1, IgG2, and IgG4), the half-lives of fusion proteins, comprising a polypeptide fused to the Fc portion of an immunoglobulin molecule, are usually in the order of days, which is significantly less than most antibodies. It would, therefore, be desirable to use fusions with intact antibodies to extend the half-lives of fast clearing peptides or polypeptides. However, fusions to termini or intact antibodies pose potentially serious problems because of off target binding by the antibody scaffold. There is a need for creating ways of extending the plasma half-lives of peptides and polypeptides to match the half-lives of native immunoglobulin molecules, without the danger of off-target binding or other deleterious side-effects.

In other situations it might be desirable to shorten the half-life of a biologically active molecule, such as a toxic molecule, or a sleeping aid, which should clear from the body after a certain time period (e. g. 6 to 8 hours) in order to avoid grogginess due to the effect of the remaining drug in the circulation during waking hours. Modulation of the pharmacokinetic properties of a biologically active molecule might also be desirable to avoid or minimize undesirable drug-drug interactions.
WO2008/036449 describes long-lived recombinant fusions of GLP-I to either the C-terminus of the VpreB 1 tail or the N-terminal tail of λ5.

### Summary of the Invention

The present invention is based, at least in part, on the finding that the pharmacokinetic properties of biologically active moieties, such as peptides and polypeptides, or associated peptidic and non-peptidic molecules, can be modulated by conjugation to a functionally null scaffold. Thus, for example, the *in vivo* half-lives of rapidly clearing peptides and polypeptides, or secondarily associated peptidic and non-peptidic molecules, can be extended by conjugation to a longer half-life functionally null scaffold, such as, for example, a functionally null antibody, Surrobody™ (hereinafter referred to as "Surrobody") or other scaffold comprising a functionally null binding region, such as an Adnectin™ (hereinafter referred to as "Adnectin"), Domain Antibody™ (hereinafter referred to as "Domain Antibody" or "dAB"), DARPin, anti-calin, Affibody, or fragments thereof.

In one aspect, the invention concerns a fusion molecule comprising a first moiety and a second moiety, wherein said second moiety comprises one or more functionally null binding regions fused to and capable of modulating at least one pharmacokinetic property of the first moiety, as defined in the claims.

In yet another aspect, the disclosure concerns a composition comprising a fusion molecule herein, in admixture with a pharmaceutically acceptable excipient.

In a further aspect, the disclosure concerns a method of modulating a pharmacokinetic property of a molecule comprising conjugating such molecule to a moiety comprising at least one functionally null binding region.

In a still further aspect, the disclosure concerns the use of a fusion molecule herein to modulate a pharmacokinetic property of a molecule or another moiety.

In another embodiment, the biologically active moiety is a peptide or a polypeptide.

In yet another embodiment, the biologically active moiety and the moiety comprising one or more functionally null binding regions are fused to each other.

The pharmacokinetic property is *in vivo* half-life.

The one or more functionally null binding regions extends the *in vivo* half-life of the biologically active moiety to which it is conjugated.

The moiety comprising one or more functionally null binding regions is a Surrobody or a fragment thereof.

Various further specific embodiments are disclosed in the rest of the specification and in the claims.

### Brief Description of the Drawings

Figure 1 shows the human VpreB1 amino acid sequence of SEQ ID NO: 1; the mouse VpreB2 sequences of SEQ ID NOS: 2 and 3; the human VpreB3 sequence of SEQ ID NO: 4, the human λ5 sequence of SEQ D NO: 5 and the human λ5-like protein sequence of SEQ ID NO: 6, and sequences of various Surrobody constructs.
Figure 2 is a schematic illustration of a surrogate light chain formed by VpreB and λ5 sequences, illustrative fusion polypeptides comprising surrogate light chain sequences, and an antibody light chain structure derived from V-J joining.
Figure 3 is a schematic illustration of various surrogate light chain deletion and single chain constructs.
Figure 4 schematically illustrates the incorporation of combinatorial functional diversity into surrogate light chain constructs. The short, thinner segments below the polypeptide segments depicted as solid white and hatched bars represent appended diversity such as a peptide library.
Figure 5 shows the gene and protein structures of various illustrative surrogate light chain constructs.
Figure 6 illustrates various representative ways of adding functionality to surrogate light chain (SLC) components.
Figure 7 illustrates various trimeric and dimeric surrogate light chain (SLC) constructs.
Figure 8 schematically illustrates various sites of incorporation for chimeric antibody-based fusion molecules of the present invention.
Figure 9 schematically illustrates various sites of incorporation for chimeric VpreB and λ5 molecules of the present invention.
Figure 10 schematically illustrates various sites of incorporation for chimeric VpreB-λ5 fusion proteins of the present invention.
Figure 11 is a schematic illustration of various heterodimeric surrogate κ light chain deletion variants. In the "full length" construct, both the Vκ-like and JCκ sequence retains the C- and N-terminal extensions (tails), respectively. In the dJ variant, the N-terminal extension of JCκ has been deleted. In the dVκ tail variants, the C-terminal extension of the Vκ-like sequence had been removed but the N-terminal extension of JCκ is retained. In the "short kappa" variant, both the C-terminal tail of the Vκ-like sequence and the N-terminal extension of the JCκ sequence are retained.
Figure 12: κ-like light chain deletion and single chain constructs, which can be used individually or with another protein, such as an antibody heavy chain or a fragment thereof.
Figure 13: Mature GLP-1 Ser8 SLC sequences.
Figure 14: Serum Stability of GLP-1 two-piece S2g Surrobody.
Figure 15: Serum Stability of GLP-1 three-piece S3g Surrobody.
Figure 16: GLP-1 Surrobodies activate stable GLP-1 receptor reporter cells.
Figure 17: GLP-1 Sg Reduces Blood Glucose thru 8 hours in vivo.
Figure 18: Exendin-4 Surrobodies Maintain Full Potency and Efficacy in vitro.

### Detailed Description of the Invention

### A. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), provides one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

Throughout this application, the use of singular includes the plural unless expressly stated otherwise.

In this application, the use of "or" includes "and/or", unless expressly stated otherwise.

Furthermore, the terms, "include," "including," and "included," are not limiting.

The term "pharmacokinetic property" is used herein to refer to a parameter that describes the disposition of an active agent in an organism or host. Representative pharmacokinetic properties include *in vivo* (plasma) half-life, clearance, rate of elimination; volume of distribution, degree of tissue targeting, degree of cell type targeting, and the like.

The terms "half-life" *"in vivo* half-life" and "plasma half-life" are used interchangeably, and refer to the time by which half of the administered amount of a molecule, such as a peptide or polypeptide, is removed from the blood stream.

The terms "clearance rate" and "clearance" refer to the rate at which a molecule, such as a peptide or polypeptide, is removed from the blood stream.

By "volume of distribution" is meant the distribution and degree of retention of a drug throughout the various compartments of an organism, e.g. intracellular and extracellular spaces, tissues and organs.

The term "moiety" is used herein in the broadest sense and including a molecule or part of a molecule.

The term "biologically active moiety" is used herein in the broadest sense to refer to any molecule or a fragment thereof that is capable of affecting a biological process in a living organism, such as a human or a non-human animal. The term specifically includes drug moieties, including polypeptides, peptides, non-peptide small organic molecules, and fragments thereof, whether derived from natural sources and/or produced by synthetic means, regardless of the indication, target disease or condition, or mechanism of action.

The term "pharmacokinetic modulating moiety" is used herein to refer to a moiety capable of modulating at least one pharmacokinetic property of a biologically active moiety to which it is conjugated. The pharmacokinetic modulating moiety of the present invention is a moiety comprising at least one functionally null binding region.

The term "functionally null binding region" within a pharmacokinetic modulating moiety, refers to a binding region, which, following administration of the pharmacokinetic modulating moiety to a recipient living organism, does not appreciably bind to any self-target or foreign target present in the recipient living organism. The definition specifically includes a binding region that has binding affinity for a self- or foreign target present in the living organism but such self- or foreign is inaccessible to the binding region. In addition, the definition specifically includes a binding region that has binding affinity for a self- or foreign target, but such target is not present in the recipient living organism.

Pharmacokinetic modulating moieties comprising at least one functionally null binding region specifically include binding or targeting molecules with a scaffold that includes one or more binding (targeting) regions, which do not appreciably bind to any self-target or foreign target present in the body of the recipient, and fragments thereof. This definition specifically includes, without limitation, functionally null antibodies, Surrobodies, Adnectins, dABs, DARPins, anti-calins, and Affibodies, and fragments thereof.

The biologically active moieties and the pharmacokinetic modulating moieties present in the conjugates of the present invention are not associated with each other in nature, at least not in the form in which they are present in the conjugates herein.

The terms "conjugate," "conjugated," and "conjugation" refer to any and all forms of covalent or non-covalent linkage, and include, without limitation, direct genetic or chemical fusion, coupling through a linker or a cross-linking agent, and non-covalent association, for example through Van der Waals forces, or by using a leucine zipper.

The term "flexible linker" is used herein to refer to any linker that is not predicted, based on its chemical structure, to be fixed in three-dimensional space in its intended context and environment.

The term "fusion" is used herein to refer to the combination of amino acid sequences of different origin in one polypeptide chain by in-frame combination of their coding nucleotide sequences. The term "fusion" explicitly encompasses internal fusions, i.e., insertion of sequences of different origin within a polypeptide chain, in addition to fusion to one of its termini.

In the context of the present invention, the term "antibody" (Ab) is used to refer to a native antibody from a classically recombined heavy chain derived from V(D)J gene recombination and a classically recombined light chain also derived from VJ gene recombination, or a fragment thereof.

A "native antibody" is heterotetrameric glycoprotein of about 150,000 daltons, composed of two identical light (L) chains and two identical heavy (H) chains. Each light chain is linked to a heavy chain by covalent disulfide bond(s), while the number of disulfide linkages varies between the heavy chains of different immunoglobulin isotypes. Each heavy and light chain also has regularly spaced intrachain disulfide bridges. Each heavy chain has, at one end, a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light- and heavy-chain variable domains, Chothia et al., J. Mol. Biol. 186:651 (1985); Novotny and Haber, Proc. Natl. Acad. Sci. U.S.A. 82:4592 (1985).

The term "variable" with reference to antibody chains is used to refer to portions of the antibody chains which differ extensively in sequence among antibodies and participate in the binding and specificity of each particular antibody for its particular antigen. Such variability is concentrated in three segments called hypervariable regions both in the light chain and the heavy chain variable domains. The more highly conserved portions of variable domains are called the framework region (FR). The variable domains of native heavy and light chains each comprise four FRs (FR1, FR2, FR3 and FR4, respectively), largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, Md. (1991), pages 647-669). The constant domains are not involved directly in binding an antibody to an antigen, but exhibit various effector functions, such as participation of the antibody in antibody-dependent cellular toxicity.

The term "hypervariable region" when used herein refers to the amino acid residues of an antibody which are responsible for antigen-binding. The hypervariable region comprises amino acid residues from a "complementarity determining region" or "CDR" (*i.e.,* residues 30-36 (L1), 46-55 (L2) and 86-96 (L3) in the light chain variable domain and 30-35 (H1), 47-58 (H2) and 93-101 (H3) in the heavy chain variable domain; MacCallum et al,. J Mol Biol. 262(5):732-45 (1996).

The term "framework region" refers to the art recognized portions of an antibody variable region that exist between the more divergent CDR regions. Such framework regions are typically referred to as frameworks 1 through 4 (FR1, FR2, FR3, and FR4) and provide a scaffold for holding, in three-dimensional space, the three CDRs found in a heavy or light chain antibody variable region, such that the CDRs can form an antigen-binding surface.

Depending on the amino acid sequence of the constant domain of their heavy chains, antibodies can be assigned to different classes. There are five major classes of antibodies IgA, IgD, IgE, IgG, and IgM, and several of these may be further divided into subclasses (isotypes), e.g., IgG1, IgG2, IgG3, IgG4, IgA, and IgA2. In a preferred embodiment, the immunoglobulin sequences used in the construction of the immunoadhesins of the present invention are from an IgG immunoglobulin heavy chain domain. For human immunoadhesins, the use of human IgG1 and IgG3 immunoglobulin sequences is preferred. A major advantage of using the IgG1 is that IgG1 immunoadhesins can be purified efficiently on immobilized protein A. However, other structural and functional properties should be taken into account when choosing the Ig fusion partner for a particular immunoadhesin construction. For example, the IgG3 hinge is longer and more flexible, so that it can accommodate larger "adhesin" domains that may not fold or function properly when fused to IgG1. Another consideration may be valency; IgG immunoadhesins are bivalent homodimers, whereas Ig subtypes like IgA and IgM may give rise to dimeric or pentameric structures, respectively, of the basic Ig homodimer unit. For VEGF receptor Ig-like domain/immunoglobulin chimeras designed for in vivo applications, the pharmacokinetic properties and the effector functions specified by the Fc region are important as well. Although IgG1, IgG2 and IgG4 all have in vivo half-lives of 21 days, their relative potencies at activating the complement system are different. Moreover, various immunoglobulins possess varying numbers of allotypic isotypes.

The heavy-chain constant domains that correspond to the different classes of immunoglobulins are called α, δ, ε, γ, and µ, respectively.

The "light chains" of antibodies from any vertebrate species can be assigned to one of two clearly distinct types, called kappa (κ) and lambda (λ), based on the amino acid sequences of their constant domains. Any reference to an antibody light chain herein includes both κ and λ light chains.

"Antibody fragments" comprise a portion of a full length antibody, generally the antigen binding or a variable domain thereof. Examples of antibody fragments include, but are not limited to, Fab, Fab', F(ab')₂, scFv, and (scFv)₂ fragments.

As used herein the term "antibody binding region" refers to one or more portions of an immunoglobulin or antibody variable region capable of binding an antigen(s). Typically, the antibody binding region is, for example, an antibody light chain (VL) (or variable region thereof), an antibody heavy chain (VH) (or variable region thereof), a heavy chain Fd region, a combined antibody light and heavy chain (or variable region thereof) such as a Fab, F(ab')₂, single domain, or single chain antibody (scFv), or a full length antibody, for example, an IgG *(e.g.,* an IgG1, IgG2, IgG3, or IgG4 subtype), IgA1, IgA2, IgD, IgE, or IgM antibody.

The term "epitope" as used herein, refers to a sequence of at least about 3 to 5, preferably at least about 5 to 10, or at least about 5 to 15 amino acids, and typically not more than about 500, or about 1,000 amino acids, which define a sequence that by itself, or as part of a larger sequence, binds to an antibody generated in response to such sequence. An epitope is not limited to a polypeptide having a sequence identical to the portion of the parent protein from which it is derived. Indeed, viral genomes are in a state of constant change and exhibit relatively high degrees of variability between isolates. Thus the term "epitope" encompasses sequences identical to the native sequence, as well as modifications, such as deletions, substitutions and/or insertions to the native sequence. Generally, such modifications are conservative in nature but non-conservative modifications are also contemplated. The term specifically includes "mimotopes," i.e. sequences that do not identify a continuous linear native sequence or do not necessarily occur in a native protein, but functionally mimic an epitope on a native protein. The term "epitope" specifically includes linear and conformational epitopes.

The term "surrogate light chain," as used herein, refers to a dimer formed by the non-covalent association of a VpreB and a λ5 protein or a "κ-like" surrogate light chain.

The term "surrogate light chain sequence," as defined herein, means any polypeptide sequence that comprises a (1) "VpreB sequence" and/or a "λ5 sequence," and/or (2) a "Vκ-like" and/or a "JCκ sequence." Surrogate light chain sequences comprising a Vκ-like and/or a JCκ sequence are also referred to as "κ-like surrogate light chain sequence." Specifically included within the definition are sequences which include both VpreB/λ5 and κ-like sequences.

The "surrogate light chain sequence" comprising a VpreB sequence and/or a λ5 sequence, specifically includes, without limitation, the human VpreB 1 sequence of SEQ ID NO 1, the mouse VpreB2 sequences of SEQ ID NOs: 2 and 3, and the human VpreB3 sequence of SEQ ID NO: 4, and/or the human λ5 sequence of SEQ ID NO: 5, the human λ5-like sequence of SEQ ID NO: 6, and isoforms, including splice variants and variants formed by posttranslational modifications, homologues in other mammalian species, as well as fragments and variants of such VpreB and/or λ5 sequences, and structures comprising such sequences.

The "surrogate light chain sequence" comprising a Vκ-like and/or a JCκ sequence, specifically includes, without limitation, the human Vκ-like polypeptide AJ004956 (SEQ ID NO: 7) or any of the Vκ-like polypeptides of SEQ ID NOs: 8-19, and/or any of the AAB32987 human JCκ polyepeptide of SEQ ID NO: 20 and the JCκ-like polypeptides of SEQ ID NOs: 21-25, and structures comprising such sequences.

The term "VpreB" is used herein in the broadest sense and refers to any native sequence or variant VpreB polypeptide, specifically including, without limitation, human VpreB 1 of SEQ ID NO: 1, mouse VpreB2 of SEQ ID NOS: 2 and 3, human VpreB3 of SEQ ID NO: 4 and isoforms, including splice variants and variants formed by posttranslational modifications, other mammalian homologues thereof, as well as fragments and variants of such native sequence polypeptides.

The term "λ5" is used herein in the broadest sense and refers to any native sequence or variant λ5 polypeptide, specifically including, without limitation, human λ5 of SEQ ID NO: 5, human λ5-like protein of SEQ ID NO: 6, and their isoforms, including splice variants and variants formed by posttranslational modifications, other mammalian homologous thereof, as well a variants of such native sequence polypeptides.

The terms "variant VpreB polypeptide" and "a variant of a VpreB polypeptide" are used interchangeably, and are defined herein as a polypeptide differing from a native sequence VpreB polypeptide at one or more amino acid positions as a result of an amino acid modification. The "variant VpreB polypeptide," as defined herein, will be different from a native antibody λ or κ light chain sequence, or a fragment thereof. The "variant VpreB polypeptide" will preferably retain at least about 65%, or at least about 70%, or at least about 75%, or at least about 80%, or at least about 85%, or at least about 90%, or at least about 95%, or at least about 98% sequence identity with a native sequence VpreB polypeptide. In another preferred embodiment, the "variant VpreB polypeptide" will be less than 95%, or less than 90%, or less than 85%, or less than 80%, or less than 75%, or less than 70%, or less than 65%, or less than 60% identical in its amino acid sequence to a native antibody λ or κ light chain sequence. Variant VpreB polypeptides specifically include, without limitation, VpreB polypeptides in which the non-Ig-like unique tail at the C-terminus of the VpreB sequence is partially or completely removed.

The terms "variant λ5 polypeptide" and "a variant of a λ5 polypeptide" are used interchangeably, and are defined herein as a polypeptide differing from a native sequence λ5 polypeptide at one or more amino acid positions as a result of an amino acid modification. The "variant λ5 polypeptide," as defined herein, will be different from a native antibody λ or κ light chain sequence, or a fragment thereof. The "variant λ5 polypeptide" will preferably retain at least about 65%, or at least about 70%, or at least about 75%, or at least about 80%, or at least about 85%, or at least about 90%, or at least about 95%, or at least about 98% sequence identity with a native sequence λ5 polypeptide. In another preferred embodiment, the "variant λ5 polypeptide" will be less than 95%, or less than 90%, or less than 85%, or less than 80%, or less than 75%, or less than 70%, or less than 65%, or less than 60% identical in its amino acid sequence to a native antibody λ or κ light chain sequence. Variant λ5 polypeptides specifically include, without limitation, λ5 polypeptides in which the unique tail at the N-terminus of the λ5 sequence is partially or completely removed.

The term "VpreB sequence" is used herein to refer to the sequence of "VpreB," as hereinabove defined, or a fragment thereof.

The term "λ5 sequence" is used herein to refers to the sequence of "λ5," as hereinabove defined, or a fragment thereof.

The terms "κ-like surrogate light chain variable domain," "Vκ-like SLC," and "Vκ-like" are used interchangeably, and refer to any native sequence polypeptide that is the product of an unrearranged Vκ gene, and variants thereof. Native sequence "Vκ-like" polypeptides specifically include, without limitation, the human κ-like polypeptide AJ004956 of SEQ ID NO: 7; and the human Vκ-like polypeptides of SEQ ID NOs: 8-19, as well as homologs in non-human mammalian species, in particular species which, like humans, generate antibody diversity predominantly by gene rearrangement and/or hypermutation, such as rodents, e.g. mice and rats, and non-human higher primates. In one embodiment, variants of native sequence Vκ-like polypeptides comprise a C-terminal extension (tail) relative to antibody κ light chain sequences. In a particular embodiment, variants of native sequence Vκ-like polypeptides retain at least part, and preferably all, of the unique C-terminal extension (tail) that distinguishes the Vκ-like polypeptides from the corresponding antibody κ light chains. In another embodiment, the C-terminal tail of the variant Vκ-like polypeptide is a sequence not naturally associated with the rest of the sequence. In the latter embodiment, the difference between the C-terminal tail naturally present in the native Vκ-like sequence and the variant sequence may result from one or more amino acid alterations (substitutions, insertions, deletions, and/or additions), or the C-terminal tail may be identical with a tail present in nature in a different Vκ-like protein. Thus, for example, in any of the Vκ-like proteins of SEQ ID NOs: 7-19, and the C-terminal extension may be replaced by the C-terminal extension of another Vκ-like protein and/or altered so that it differs from any naturally occurring C-terminal extension sequence. Alternatively or in addition, variants of native sequence Vκ-like polypeptides may contain one or more amino acid alterations in the part of the sequence that is identical to a native antibody κ variable domain sequence, in particular in one or more of the complementarity determining regions (CDRs) and/or framework residues of such sequence. Thus, the Vκ-like polypeptides may contain amino acid alterations in regions corresponding to one or more of antibody κ light chain CDR1, CDR2 and CDR3 sequences. In all instances, the variants can, and preferably do, include a C-terminal extension of at least four, or at least five, or at least six, or at least seven, or at least eight, or at least nine, or at least ten amino acids, preferably 4-100, or 4-90, or 4-80, or 4-70, or 4-60, or 4-50, or 4-45, or 4-40, or 4-35, or 4-30, or 4-25, or 4-20, or 4-15, or 4-10 amino acid residues relative to a native antibody κ light chain variable region sequence. As defined herein, Vκ-like polypeptide variant will be different from a native antibody κ or λ light chain sequence or a fragment thereof, and will preferably retain at least about 65%, or at least about 70%, or at least about 75%, or at least about 80%, or at least about 85%, or at least about 90%, or at least about 95%, or at least about 98% sequence identity with a native sequence Vκ polypeptide. In another preferred embodiment, the Vκ-like polypeptide variant will be less than 95%, or less than 90%, or less than 85%, or less than 80%, or less than 75%, or less than 70%, or less than 65%, or less than 60%, or less than 55%, or less than 50%, or less than 45%, or less than 40% identical in its amino acid sequence to a native antibody λ or κ light chain sequence. In other embodiments, the sequence identity is between about 40% and about 95%, or between about 45% and about 90%, or between about 50% and about 85%, or between about 55% and about 80%, or between about 60 % and about 75%, or between about 60% and about 80%, or between about 65% and about 85%, or between about 65% and about 90%, or between about 65% and about 95%. In all embodiments, preferably the Vκ-like polypeptides are capable of binding to a target.

The terms "JCκ" and "JCκ-like" are used interchangeably, and refer to native sequence polypeptides that include a portion identical to a native sequence κ J-constant (C) region segment and a unique N-terminal extension (tail), and variants thereof. Native sequence JCκ-like polypeptides include, without limitation, the AAB32987 human JCκ polyepeptide of SEQ ID NO: 20 and the JCκ-like polypeptides of SEQ ID NOs: 21-25, as well as homologs in non-human mammalian species, in particular species which, like humans, generate antibody diversity predominantly by gene rearrangement and/or hypermutation, such as rodents, e.g. mice and rats, and non-human higher primates. In one embodiment, variants of native sequence JCκ-like polypeptides comprise an N-terminal extension (tail) that distinguishes them from an antibody JC segment. In a particular embodiment, variants of native sequence JCκ-like polypeptides retain at least part, and preferably all, of the unique N-terminal extension (tail) that distinguishes the JCκ-like polypeptides from the corresponding antibody κ light chain JC segments. In another embodiment, the N-terminal tail of the variant JCκ-like polypeptide is a sequence not naturally associated with the rest of the sequence. In the latter embodiment, the difference between the N-terminal tail naturally present in the native JCκ-like sequence and the variant sequence may result from one or more amino acid alterations (substitutions, insertions, deletions, and/or additions), or the N-terminal tail may be identical with a tail present in nature in a different JCκ-like protein. Thus, for example, in any of the JCκ-like proteins, the N-terminal extension may be replaced by the N-terminal extension of another JCκ-like protein and/or altered so that it differs from any naturally occurring N-terminal extension sequence. Alternatively or in addition, variants of native sequence JCκ-like polypeptides may contain one or more amino acid alterations in the part of the sequence that is identical to a native antibody κ variable domain JC sequence. In all instances, the variants can, and preferably do, include an N-terminal extension (unique N-terminus) of at least four, or at least five, or at least six, or at least seven, or at least eight, or at least nine, or at least ten amino acids, preferably 4-100, or 4-90, or 4-80, or 4-70, or 4-60, 4-50, or 4-45, or 4-40, or 4-35, or 4-30, or 4-25, or 4-20, or 4-15, or 4-10 amino acid residues relative to a native antibody κ light chain JC sequence. The JCκ-like polypeptide variant, as defined herein, will be different from a native antibody λ or κ light chain JC sequence, or a fragment thereof, and will preferably retain at least about 65%, or at least about 70%, or at least about 75%, or at least about 80%, or at least about 85%, or at least about 90%, or at least about 95%, or at least about 98% sequence identity with a native sequence JC polypeptide. In another preferred embodiment, the JCκ-like polypeptide variant will be less than 95%, or less than 90%, or less than 85%, or less than 80%, or less than 75%, or less than 70%, or less than 65%, or less than 60% identical in its amino acid sequence to a native antibody λ or κ light chain JC sequence. In other embodiments, the sequence identity is between about 40% and about 95%, or between about 45% and about 90%, or between about 50% and about 85%, or between about 55% and about 80%, or between about 60 % and about 75%, or between about 60% and about 80%, or between about 65% and about 85%, or between about 65% and about 90%, or between about 65% and about 95%.

The "κ-like" surrogate light chain sequence may be optionally conjugated to a heterogeneous amino acid sequence, or any other heterogeneous component, to form a "κ-like surrogate light chain construct" herein. Thus, the term, "κ-like surrogate light chain construct" is used in the broadest sense and includes any and all additional heterogeneous components, including a heterogeneous amino acid sequence, nucleic acid, and other molecules conjugated to a κ-like surrogate light chain sequence, wherein "conjugation" is defined below. In a preferred embodiment, the "κ-like surrogate light chain sequence" is capable of binding to a target. In a preferred embodiment, the "κ-like" surrogate light chain sequence is non-covalently or covalently associated with a JCκ-like sequence and/or an antibody heavy chain sequence or a fragment thereof. Covalent association includes direct fusions but also connection through a linker. Thus, for example, the Vκ-like and JCκ-like sequences may be connected via antibody light and/or heavy chain variable region sequences.

Percent amino acid sequence identity may be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

The terms "Surrobody" and "surrogate light chain construct" are used interchangeably and refer to any construct comprising a surrogate light chain sequence, and may include any and all additional heterogeneous components, including a heterogeneous amino acid sequence, nucleic acid, and other molecules conjugated to a surrogate light chain sequence. Certain Surrobody constructs are disclosed in Xu et al., Proc. Natl. Acad. Sci. USA 2008, 105(31):10756-61 and in PCT Publication WO 2008/118970 published on October 2, 2008.

In the context of the polypeptides of the present invention, the term "heterogeneous amino acid sequence relative to another (first) amino acid sequence is used to refer to an amino acid sequence not naturally associated with the first amino acid sequence, at least not in the form it is present in the particular new construct. Thus, a "heterogenous amino acid sequence" relative to a VpreB is any amino acid sequence not associated with native VpreB in its native environment, including, without limitation, λ5 sequences that are different from those λ5 sequences that, together with VpreB, form the surrogate light chain on developing B cells, such as amino acid sequence variants, e.g. truncated and/or derivatized λ5 sequences. A "heterogeneous amino acid sequence" relative to a VpreB also includes λ5 sequences covalently associated with, e.g. fused to, VpreB, including native sequence λ5, since in their native environment, the VpreB and λ5 sequences are not covalently associated, e.g. fused, to each other. Heterogeneous amino acid sequences also include, without limitation, antibody sequences, including antibody and heavy chain sequences and fragments thereof, such as, for example, antibody light and heavy chain variable region sequences, and antibody light and heavy chain constant region sequences.

As used herein the term "agonist" refers to a biologically active ligand which binds to its complementary biologically active receptor activating the receptor to induce a biological response in the receptor, or to enhance the preexisting biological activity of the receptor.

As used herein, the terms "peptide," "polypeptide" and "protein" all refer to a primary sequence of amino acids that are joined by covalent "peptide linkages." In general, a peptide consists of a few amino acids, typically from about 2 to about 50 amino acids, and is shorter than a protein. The term "polypeptide," as defined herein, encompasses peptides and proteins.

The term "amino acid" or "amino acid residue" typically refers to an amino acid having its art recognized definition such as an amino acid selected from the group consisting of: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val) although modified, synthetic, or rare amino acids may be used as desired. Thus, modified and unusual amino acids listed in 37 CFR 1.822(b)(4) are specifically included within this definition and expressly incorporated herein by reference. Amino acids can be subdivided into various sub-groups. Thus, amino acids can be grouped as having a nonpolar side chain *(e.g.,* Ala, Cys, Ile, Leu, Met, Phe, Pro, Val); a negatively charged side chain (*e.g.,* Asp, Glu); a positively charged side chain *(e.g.,* Arg, His, Lys); or an uncharged polar side chain (*e.g*., Asn, Cys, Gln, Gly, His, Met, Phe, Ser, Thr, Trp, and Tyr). Amino acids can also be grouped as small amino acids (Gly, Ala), nucleophilic amino acids (Ser, His, Thr, Cys), hydrophobic amino acids (Val, Leu, Ile, Met, Pro), aromatic amino acids (Phe, Tyr, Trp, Asp, Glu), amides (Asp, Glu), and basic amino acids (Lys, Arg).

The term "polynucleotide(s)" refers to nucleic acids such as DNA molecules and RNA molecules and analogs thereof *(e.g.,* DNA or RNA generated using nucleotide analogs or using nucleic acid chemistry). As desired, the polynucleotides may be made synthetically, e.g., using art-recognized nucleic acid chemistry or enzymatically using, *e.g.,* a polymerase, and, if desired, be modified. Typical modifications include methylation, biotinylation, and other art-known modifications. In addition, the nucleic acid molecule can be single-stranded or double-stranded and, where desired, linked to a detectable moiety.

The term "variant" with respect to a reference polypeptide refers to a polypeptide that possesses at least one amino acid mutation or modification (i.e., alteration) as compared to a native polypeptide. Variants generated by "amino acid modifications" can be produced, for example, by substituting, deleting, inserting and/or chemically modifying at least one amino acid in the native amino acid sequence.

An "amino acid modification" refers to a change in the amino acid sequence of a predetermined amino acid sequence. Exemplary modifications include an amino acid substitution, insertion and/or deletion.

An "amino acid modification at" a specified position, refers to the substitution or deletion of the specified residue, or the insertion of at least one amino acid residue adjacent the specified residue. By insertion "adjacent" a specified residue is meant insertion within one to two residues thereof. The insertion may be N-terminal or C-terminal to the specified residue.

An "amino acid substitution" refers to the replacement of at least one existing amino acid residue in a predetermined amino acid sequence with another different "replacement" amino acid residue. The replacement residue or residues may be "naturally occurring amino acid residues" (i.e. encoded by the genetic code) and selected from the group consisting of: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val). Substitution with one or more non-naturally occurring amino acid residues is also encompassed by the definition of an amino acid substitution herein.

A "non-naturally occurring amino acid residue" refers to a residue, other than those naturally occurring amino acid residues listed above, which is able to covalently bind adjacent amino acid residues(s) in a polypeptide chain. Examples of non-naturally occurring amino acid residues include norleucine, ornithine, norvaline, homoserine and other amino acid residue analogues such as those described in Ellman et al. Meth. Enzym. 202:301 336 (1991). To generate such non-naturally occurring amino acid residues, the procedures of Noren et al. Science 244:182 (1989) and Ellman et al., supra, can be used. Briefly, these procedures involve chemically activating a suppressor tRNA with a non-naturally occurring amino acid residue followed by in vitro transcription and translation of the RNA.

An "amino acid insertion" refers to the incorporation of at least one amino acid into a predetermined amino acid sequence. While the insertion will usually consist of the insertion of one or two amino acid residues, the present application contemplates larger "peptide insertions", e.g. insertion of about three to about five or even up to about ten amino acid residues. The inserted residue(s) may be naturally occurring or non-naturally occurring as disclosed above.

An "amino acid deletion" refers to the removal of at least one amino acid residue from a predetermined amino acid sequence.

The term "mutagenesis" refers to, unless otherwise specified, any art recognized technique for altering a polynucleotide or polypeptide sequence. Preferred types of mutagenesis include error prone PCR mutagenesis, saturation mutagenesis, or other site directed mutagenesis.

"Site-directed mutagenesis" is a technique standard in the art, and is conducted using a synthetic oligonucleotide primer complementary to a single-stranded phage DNA to be mutagenized except for limited mismatching, representing the desired mutation. Briefly, the synthetic oligonucleotide is used as a primer to direct synthesis of a strand complementary to the single-stranded phage DNA, and the resulting double-stranded DNA is transformed into a phage-supporting host bacterium. Cultures of the transformed bacteria are plated in top agar, permitting plaque formation from single cells that harbor the phage. Theoretically, 50% of the new plaques will contain the phage having, as a single strand, the mutated form; 50% will have the original sequence. Plaques of interest are selected by hybridizing with kinased synthetic primer at a temperature that permits hybridization of an exact match, but at which the mismatches with the original strand are sufficient to prevent hybridization. Plaques that hybridize with the probe are then selected, sequenced and cultured, and the DNA is recovered.

The term "vector" is used to refer to a rDNA molecule capable of autonomous replication in a cell and to which a DNA segment, e.g., gene or polynucleotide, can be operatively linked so as to bring about replication of the attached segment. Vectors capable of directing the expression of genes encoding for one or more polypeptides are referred to herein as "expression vectors. "The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

A "phage display library" is a protein expression library that expresses a collection of cloned protein sequences as fusions with a phage coat protein. Thus, the phrase "phage display library" refers herein to a collection of phage (e.g., filamentous phage) wherein the phage express an external (typically heterologous) protein. The external protein is free to interact with (bind to) other moieties with which the phage are contacted. Each phage displaying an external protein is a "member" of the phage display library.

The term "filamentous phage" refers to a viral particle capable of displaying a heterogenous polypeptide on its surface, and includes, without limitation, f1, fd, Pf1, and M13. The filamentous phage may contain a selectable marker such as tetracycline (e.g., "fd-tet"). Various filamentous phage display systems are well known to those of skill in the art (see, e.g., Zacher et al. Gene 9: 127-140 (1980), Smith et al. Science 228: 1315-1317 (1985); and Parmley and Smith Gene 73: 305-318 (1988)).

The term "panning" is used to refer to the multiple rounds of screening process in identification and isolation of phages carrying compounds, such as antibodies, with high affinity and specificity to a target.

The "recipient" living organism is a vertebrate human or non-human animal, preferably a mammal, more preferably a human. Mammals include, but are not limited to, humans, non-human higher primates, farm animals (such as cows), sport animals, pets (such as cats, dogs and horses), mice, and rats.

As used herein, the term "effective amount" or a "therapeutically effective amount" is the amount of a conjugate or fusion molecule, which is required to achieve a measurable improvement in the state, e.g. pathology, of the target condition, such as, for example, an insulin-related disorder.

### B. Detailed Description

Techniques for performing the methods of the present invention are well known in the art and described in standard laboratory textbooks, including, for example, Ausubel et al., Current Protocols of Molecular Biology, John Wiley and Sons (1997); Molecular Cloning: A Laboratory Manual, Third Edition, J. Sambrook and D. W. Russell, eds., Cold Spring Harbor, New York, USA, Cold Spring Harbor Laboratory Press, 2001; O'Brian et al., Analytical Chemistry of Bacillus Thuringiensis, Hickle and Fitch, eds., Am. Chem. Soc., 1990; Bacillus thuringiensis: biology, ecology and safety, T.R. Glare and M. O'Callaghan, eds., John Wiley, 2000; Antibody Phage Display, Methods and Protocols, Humana Press, 2001; and Antibodies, G. Subramanian, ed., Kluwer Academic, 2004. Mutagenesis can, for example, be performed using site-directed mutagenesis (Kunkel et al., Proc. Natl. Acad. Sci USA 82:488-492 (1985)). PCR amplification methods are described in U.S. Pat. Nos. 4,683,192, 4,683,202, 4,800,159, and 4,965,188, and in several textbooks including "PCR Technology: Principles and Applications for DNA Amplification", H. Erlich, ed., Stockton Press, New York (1989); and PCR Protocols: A Guide to Methods and Applications, Innis et al., eds., Academic Press, San Diego, Calif. (1990).

Intact immunoglobulins have long serum half-lives on the order of weeks. Removal of the Fc component of an antibody reduces the serum half-life of the resulting Fab fragment to less than one day. Typically, fusions designed to extend the half-lives of polypeptides have been constructed by fusing the polypeptide of interest to the amino terminus of the Fc portion of an antibody, usually at the hinge region. The resulting half-life is usually on the order of days, but less than the half life of most antibodies. Fusions to termini of intact antibodies have not been commonplace, and pose potential problems because of off target binding by the antibody scaffold, which retains the N-terminal antigen binding (variable region) sequences.

The present invention concerns the modulation of pharmacokinetic properties of moieties characterized by at least one unfavorable pharmacokinetic property. In particular, the invention concerns modulation of pharmacokinetic properties of biologically active moieties, including, without limitation, extension of *in vivo* half-lives of peptides, polypeptides, and secondarily associated peptidic and nonpeptidic elements. by conjugation to functionally null pharmacokinetic modulating moieties, such as, for example, antibodies, Surrobodies, Domain Antibodies (dAbs), Anectins, and fragments thereof.

### Functionally null antibodies and Surrobodies

Antibody (Ig) molecules produced by B-lymphocytes are built of heavy (H) and light (L) chains. The amino acid sequences of the amino terminal domains of the H and L chains are variable (V_{H} and V_{L}), especially at the three hypervariable regions (CDR1, CDR2, CDR3) that form the antigen combining site. The assembly of the H and L chains is stabilized by a disulfide bond between the constant region of the L chain (C_{L}) and the first constant region of the heavy chain (C_{H1}) and by non-covalent interactions between the V_{H} and V_{L} domains.

In humans and many animals, such as mice, the genes encoding the antibody H and L chains are assembled by stepwise somatic rearrangements of gene fragments encoding parts of the V regions. Various stages of B lymphocyte development are characterized by the rearrangement status of the Ig gene loci (see, e.g. Melchers, F. & Rolink, A., B-Lymphocyte Development and Biology, Paul, W.E., ed., 1999, Lippincott, Philadephia).

Surrobodies are based on the pre-B cell receptor (pre-BCR), which is produced during normal development of antibody repertoire. Unlike antibodies, pre-BCR is a trimer, that is composed of an antibody heavy chain paired with two surrogate light chain components, VpreB and λ5. Both VpreB and λ5 are encoded by genes that do not undergo gene rearrangement and are expressed in early pro-B cells before V(D)J recombination begins. The pre-BCR is structurally different from a mature immunoglobulin in that it is composed of a heavy chain and two non-covalently associated proteins: VpreB and λ5, ie they have three components as opposed to two in antibodies. Furthermore, although VpreB is homologous to the Vλ Ig domain, and λ5 is homologous to the Cλ domain of antibodies, each has noncanonical peptide extensions: VpreB 1 has additional 21 residues on its C terminus; λ5 has a 50 amino acid extension at its N terminus. Another group of Surrobodies contains a κ-like surrogate light chain (SLC) construct comprising a Vκ-like and/or a JCκ sequence, as hereinabove defined. It is also possible to construct Surrobodies that include one of more of VpreB, λ5, Vκ-like and JCκ sequences, and the use of such Surrobody constructs, and their fragments, is specifically within the scope of the present invention.

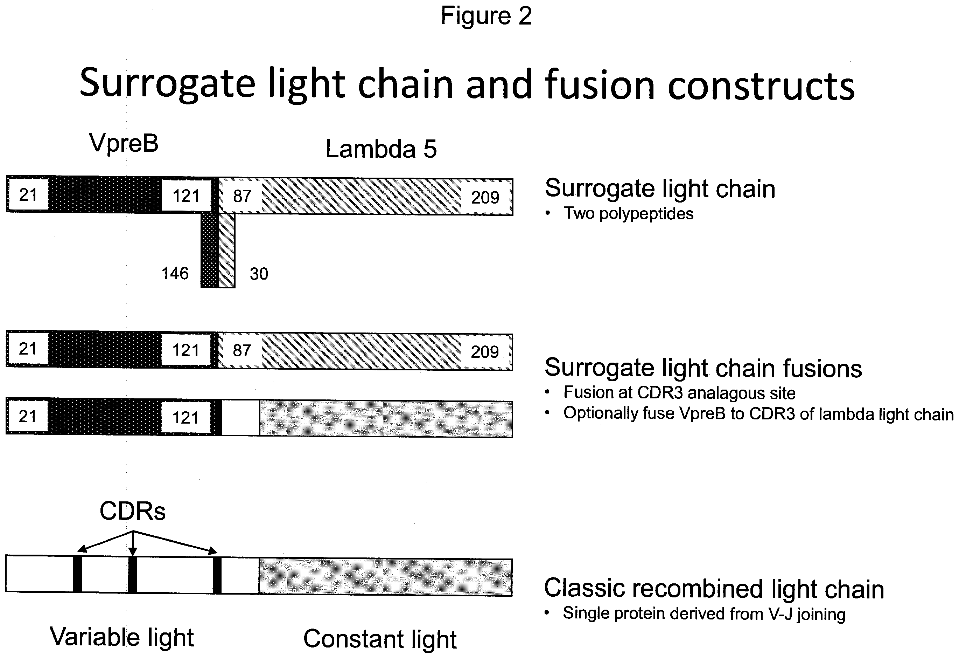

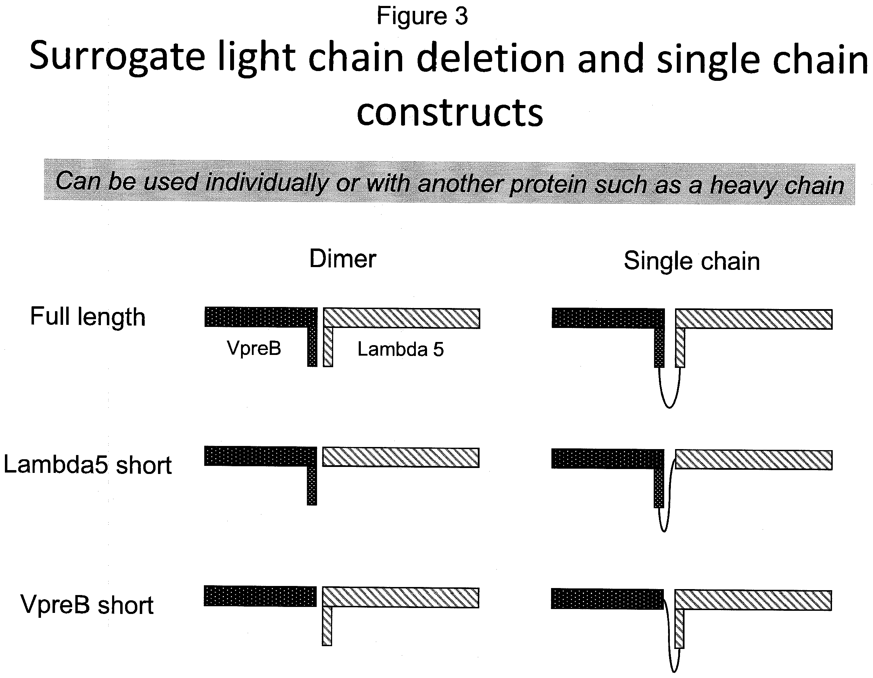

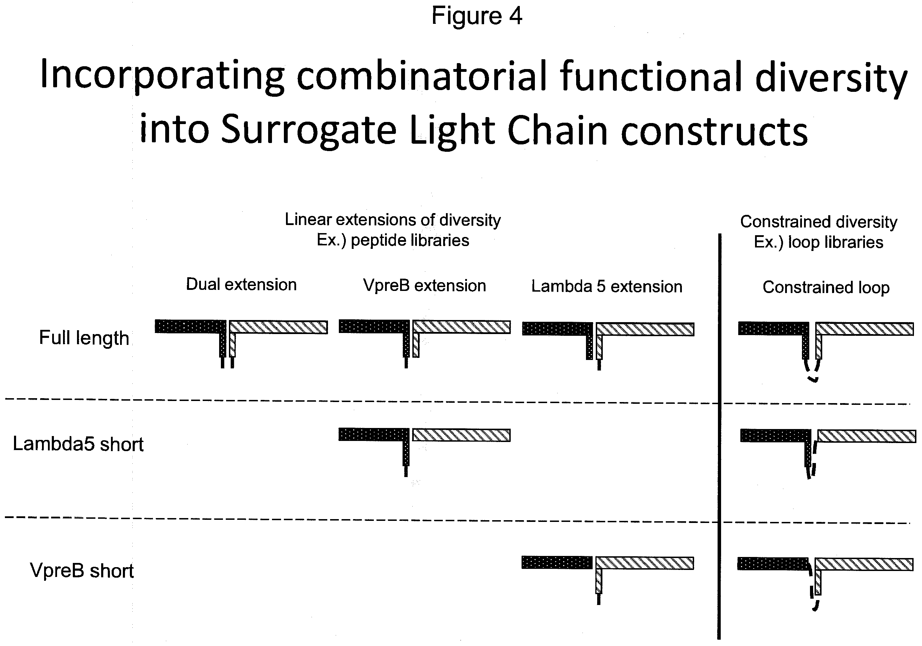

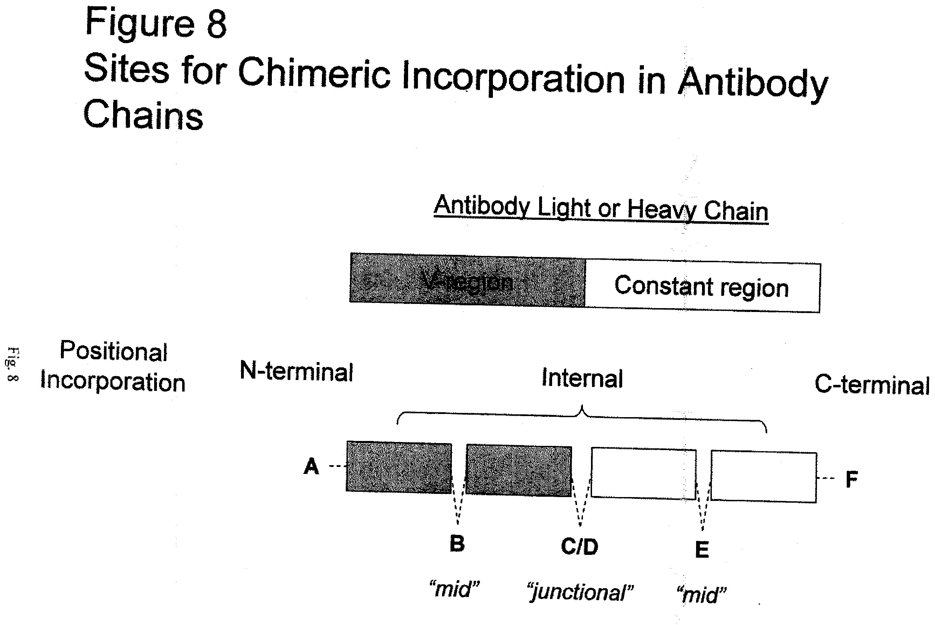

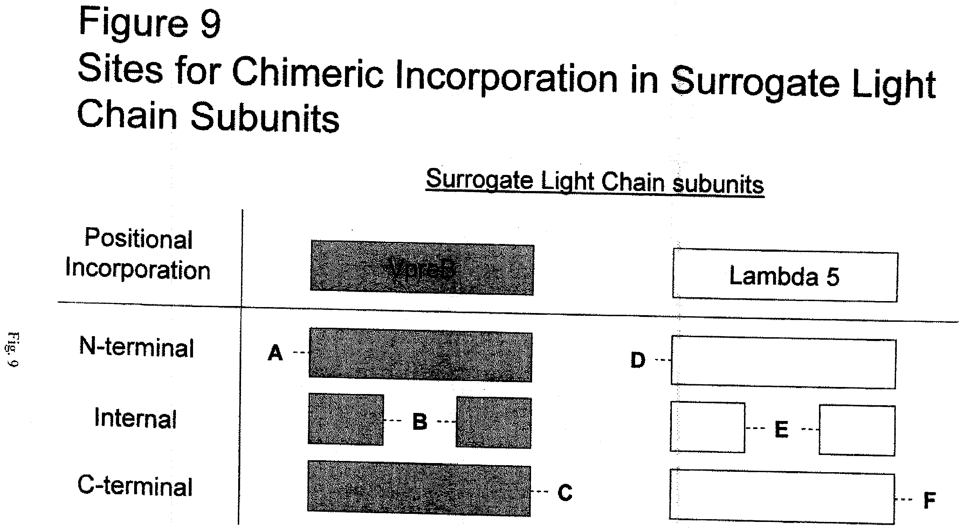

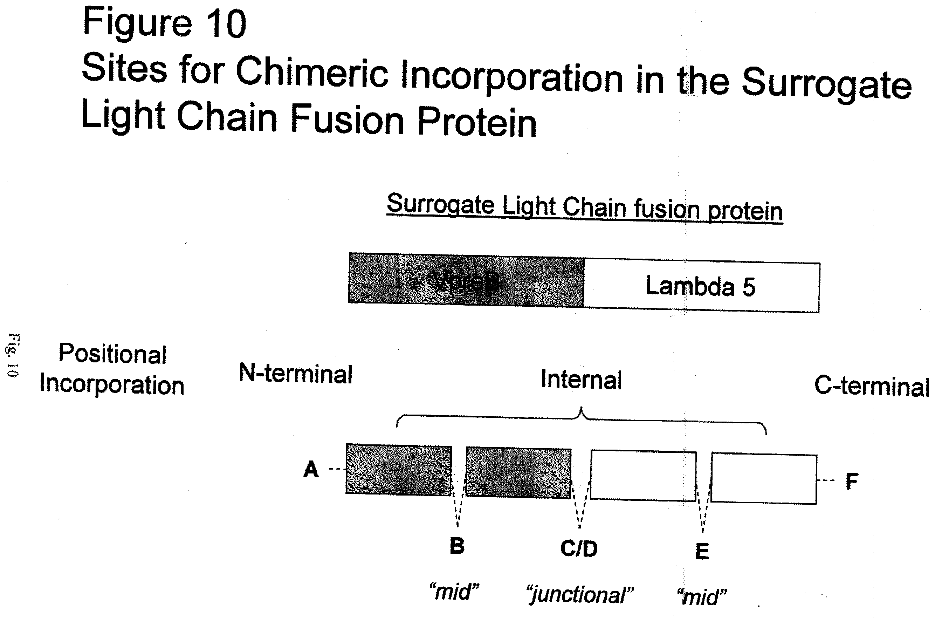

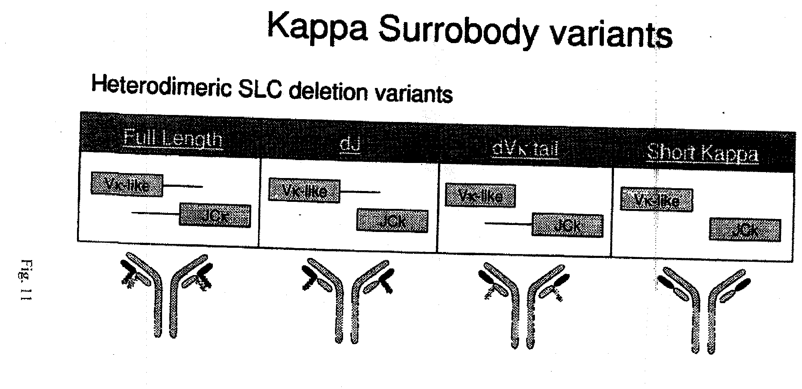

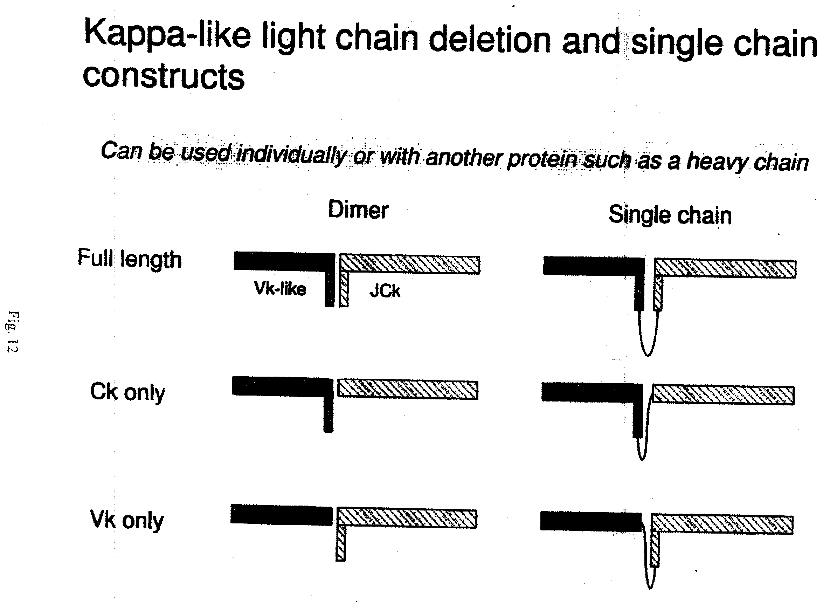

Further details of the design and production of Surrobodies are provided in Xu et al., Proc. Natl. Acad. Sci. USA 2008, 105(31):10756-61 and in PCT Publication WO 2008/118970 published on October 2, 2008. Representative Surrobody™ structures are illustrated in Figures 2-12.

Specific examples of Surrobodies include polypeptides in which a VpreB sequence, such as a VpreB 1, VpreB2, or VpreB3 sequence, including fragments and variants of the native sequences, is conjugated to a λ5 sequence, including fragments and variants of the native sequence.

In a direct fusion, typically the C-terminus of a VpreB sequence (e.g. a VpreB 1, VpreB2 or VpreB3 sequence) is fused to the N-terminus of a λ5 sequence. While it is possible to fuse the entire length of a native VpreB sequence to a full-length λ5 sequence, typically the fusion takes place at or around a CDR3 analogous site in each of the two polypeptides. One such representative fusion construct is illustrated in Figure 2. In this embodiment, the fusion may take place within, or at a location within about 10 amino acid residues at either side of the CDR3 analogous region. In a preferred embodiment, the fusion takes place between about amino acid residues 116-126 of the native human VpreB 1 sequence (SEQ ID NO: 1) and between about amino acid residues 82 and 93 of the native human λ5 sequence (SEQ ID NO: 5).

It is also possible to fuse the VpreB sequence to the CDR3 region of an antibody λ light chain. Further constructs, in which only one of VpreB and λ5 is truncated are also shown. Similar constructs can be prepared using antibody κ light chain sequences.

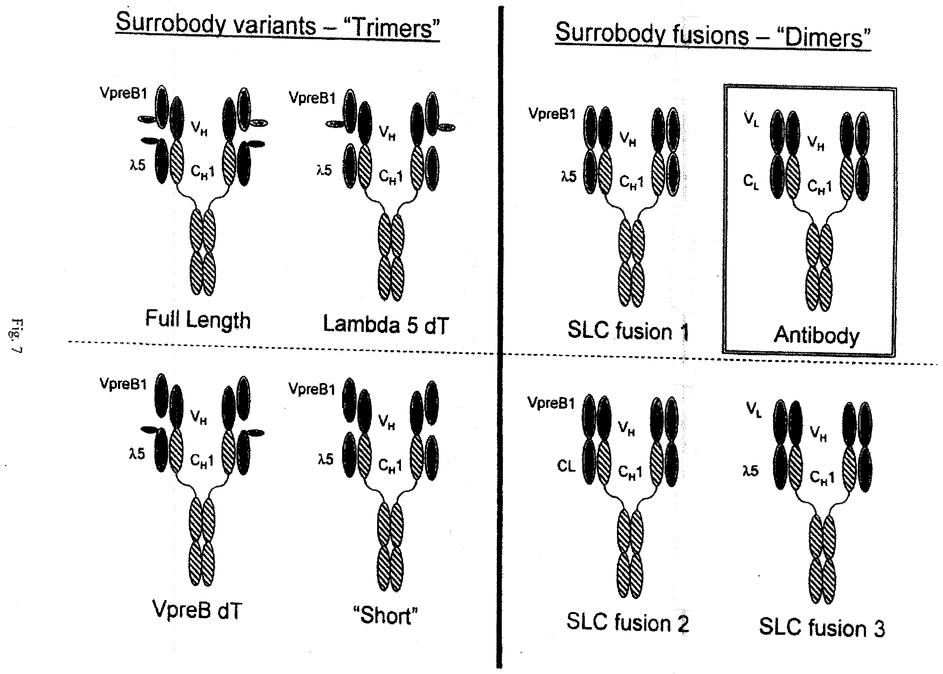

Further direct fusion structures are illustrated on the right side of Figure 7. The structure designated "SLC fusion 1" is a tetramer, composed of two dimers, in which the fusion of a truncated V-preB1 sequence (lacking the characteristic "tail" at the C-terminus of native VpreB1) to a similarly truncated λ5 sequence is non-covalently associated with an antibody heavy chain. The structure designated "SLC fusion 2" is a tetramer, composed of two dimers, in which the fusion of a truncated VpreB1 sequence (lacking the characteristic "tail" at the C-terminus of native VpreB1) to an antibody light chain constant region is non-covalently associated with an antibody heavy chain. The structure designated "SLC fusion 3" is a tetramer, composed of two dimers, in which the fusion of an antibody light chain variable region to a truncated λ5 sequence (lacking the characteristic "tail" at the N-terminus of native λ5) is non-covalently associated with an antibody heavy chain.

As noted above, in addition to direct fusions, the polypeptide constructs of the present invention include non-covalent associations of a VpreB sequence (including fragments and variants of a native sequence) with a heterogeneous sequence, such as a λ5 sequence (including fragments and variants of the native sequence), and/or an antibody sequence. Thus, for example, a full-length VpreB sequence may be non-covalently associated with a truncated λ5 sequence. Alternatively, a truncated VpreB sequence may be non-covalently associated with a full-length λ5 sequence.

Surrogate light chain constructs comprising non-covalently associated VpreB 1 and λ5 sequences, in non-covalent association with an antibody heavy chain, are shown on the left side of Figure 7. As the various illustrations show, the structures may include, for example, full-length VpreB 1 and λ5 sequences, a full-length VpreB 1 sequence associated with a truncated λ5 sequence ("Lambda 5dT"), a truncated V-reB1 sequence associated with a full-length λ5 sequence (VpreB dT") and a truncated VpreB1 sequence associated with a truncated λ5 sequence ("Short").

Although the Figures illustrate certain specific constructs, one of ordinary skill will appreciate that a variety of other constructs can be made and used in a similar fashion. For example, the structures can be asymmetrical, comprising different surrogate light chain sequences in each arm, and/or having trimeric or pentameric structures, as opposed to the structures illustrated in the Figures.

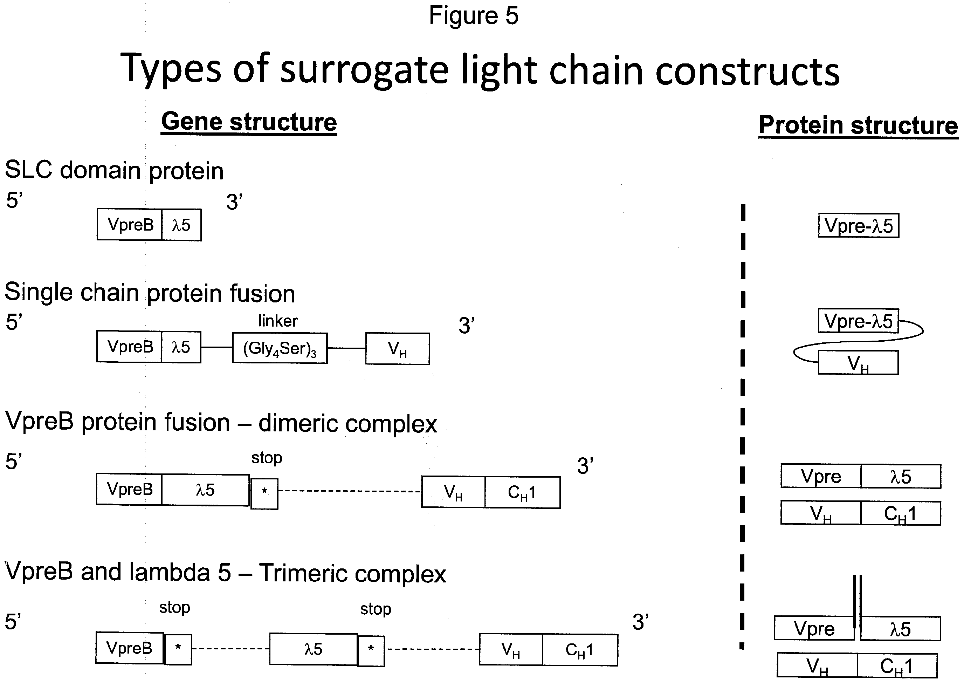

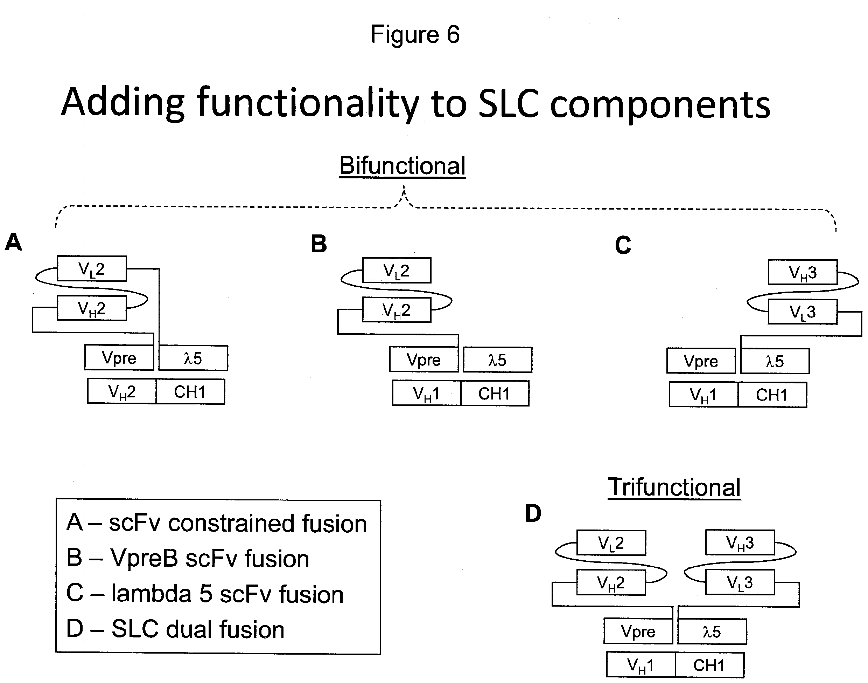

All surrogate light chain constructs (Surrobodies) herein may be associated with antibody sequences. For example, as shown in Figure 5, a VpreB-λ5 fusion can be linked to an antibody heavy chain variable region sequence by a peptide linker. In another embodiment, a VpreB-λ5 fusion is non-covalently associated with an antibody heavy chain, or a fragment thereof including a variable region sequence to form a dimeric complex. In yet another embodiment, the VpreB and λ5 sequences are non-covalently associated with each other and an antibody heavy chain, or a fragment thereof including a variable region sequence, thereby forming a trimeric complex. Exemplary constructs comprising an antibody heavy chain are illustrated in Figures 5 and 6.

Functionally null antibodies and Surrobodies can be designed and created by several strategies.

For example, a "functionally null" antibody or Surrobody can be designed to utilize antibody or Surrobody components that would not appreciably bind targets in a recipient to whom the chimeric molecules of the present invention are to be administered. On such strategy would be to use antibody or Surrobody components that recognize foreign targets include binding targets found in pathogens, provided the particular pathogen is not present in the recipient's body, or is sequestered at a location that that is inaccessible to the antibody or Surrobody. The resulting combination would not bind any antigen, avoid agglutination and removal and result in long-lived serum half-life. Other examples are targets that are inaccessible to the chimeric molecule, target and antibody Surrobody combinations with unfavorable equilibrium kinetics, or even ligand occupied molecules. Although in this definition reference is made the Surrobodies and antibodies, other "functionally null" binding molecules and binding regions are defined in a similar manner. In the broadest sense a "functionally null" binding or targeting molecule is a molecule that has one or more binding regions to a particular target, but which molecule does not appreciably bind to the target under the circumstances, regardless of the reasons for non-binding.

In one embodiment, the scaffold including one or more functionally null binding regions, such as, for example, Surrobody, antibody, dAB, Adnectin, DARPin, anti-calin, and Affibody, is specific to an inaccessible self-target, such as an intracellular or nuclear protein.

In another embodiment, antibodies are created from germline heavy and light chains from a combination of unmutated "V-J" light chain and unmutated "V-D-J" genes to encode the null antibody polypeptides. As most binding is dictated by the heavy chain CDR3 region, one the possibility of binding to any foreign or non-foreign target can be further reduced by removing the D-region or using a designed minimal D-region in creating the null antibody. Further engineering or additional deletions of portions of the V and J regions are possible to further reinforce nonreactivitiy. Similar strategies can be applied to produce functionally null Surrobodies and other moieties with functionally null binding regions.

### Conjugates comprising functionally null antibodies or Surrobodies or their fragments

The functionally null antibodies and Surrobodies are used to create conjugates, such as fusions, with the moieties which are in need of improving one or more of their pharmacokinetic properties, such as *in vivo* half-life, clearance, and the like.

Thus, for example, a peptide or polypeptide, the plasma half-life of which is to be extended, can be fused to the amino or carboxy terminus of a functionally null antibody heavy and/or light chain, or fragment thereof, retaining at least part of the antigen-binding (variable region) sequences, or incorporated into a functionally null antibody heavy and/or light chain, or a fragment thereof. The resultant fusion polypeptide is expected to have the half-life of an antibody because the risk of off-target binding and elimination is essentially avoided.

Functionally null Surrobodies, comprising a surrogate light chain construct paired with a functionally null heavy chain, can be constructed in a similar fashion, but allow more degrees of freedom, especially in the trimeric form, where the VpreB and λ5 sequences are non-covalently associated, due to the additional amino and carboxyl termini present on the two piece surrogate light chain. Additionally, it is possible to place a fusion at either end of a chimeric surrogate light chain, where the VpreB protein and λ5 protein exist as a single polypeptide.

It is also possible to fuse more than one peptide or polypeptide to the antibody or Surrobody™, and thus this method can also be used to increase the effective dose per Surrobody unit or for the simultaneous administration of multiple therapeutic proteins, while extending their plasma half-lives of each heterologous peptide or polypeptide.

In one aspect, the present disclosure provides conjugates and fusion molecules that contain a functionally null component and at least one therapeutic component having at least one pharmacokinetic property modulated by the functionally null component. In one embodiment, the conjugate includes a first moiety and a second moiety, wherein the second moiety is a scaffold comprising one or more functionally null binding regions conjugated to and capable of modulating at least one pharmacokinetic property of the first moiety, wherein the first moiety is a therapeutic moiety. In another embodiment, the fusion molecule includes a first moiety and a second moiety, wherein said second moiety comprises one or more functionally null binding regions fused to and capable of modulating at least one pharmacokinetic property of the first moiety, wherein the first moiety is a therapeutic moiety.

In another embodiment, the conjugate or fusion molecule includes more than one therapeutic moiety. The therapeutic moieties may conjugated or fused to the functionally null components as described herein. For example, a fusion molecule with therapeutic moieties peptide A and peptide B may be designed as follows: peptide A-linker-peptide A or B-linker-surrogate light chain (functionally null). Those of ordinary skill in the art will appreciate other formats that are suitable.

### Production of moieties with functionally null binding region and fusion polypeptides comprising same

Monoclonal antibodies can be prepared, e.g., using hybridoma methods, such as those described by Kohler and Milstein, Nature, 256:495 (1975) or can be made by recombinant DNA methods (U.S. Pat. Nos. 4,816,567 and 6,331,415). In a hybridoma method, a hamster, mouse, or other appropriate host animal is typically immunized with an immunizing agent to elicit lymphocytes that produce or are capable of producing antibodies that will specifically bind to the immunizing agent. Alternatively, the lymphocytes can be immunized in vitro.

The immunizing agent will typically include a target polypeptide or a fusion protein of the target polypeptide or a composition comprising the target polypeptide. As discussed above, in the present case, the target polypeptide typically is a foreign polypeptide not present in the body of the recipient, or a self-polypeptide, which is present but not available for binding in the recipient's body.

Generally, either peripheral blood lymphocytes (PBLs) are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Immortalized cell lines are usually transformed mammalian cells, particularly myeloma cells of rodent, bovine, and human origin. Usually, rat or mouse myeloma cell lines are employed. The hybridoma cells can be cultured in a suitable culture medium that preferably contains one or more substances that inhibit the growth or survival of the unfused, immortalized cells.

The culture medium in which the hybridoma cells are cultured can then be assayed for the presence of monoclonal antibodies directed against the target polypeptide by methods known in the art, such as, for example, by immunoprecipitation or by various immunoassays, such as radioimmunoassay (RIA) or enzyme-linked immunoabsorbent assay (ELISA). After the desired hybridoma cells are identified, the clones can be subcloned by limiting dilution procedures and grown by standard methods. Suitable culture media for this purpose include, for example, Dulbecco's Modified Eagle's Medium and RPMI-1640 medium. Alternatively, the hybridoma cells can be grown *in vivo* as ascites in a mammal.

The monoclonal antibodies secreted by the subclones can be isolated or purified from the culture medium or ascites fluid by conventional immunoglobulin purification procedures such as, for example, protein A or hydroxylapatite chromatography, gel electrophoresis, dialysis, or affinity chromatography.

The monoclonal antibodies can also be made by recombinant DNA methods, such as those described in U.S. Pat. Nos. 4,816,567 and 6,331,415. Surrobodies can also be produced by recombinant DNA techniques, as described, for example, in WO 2008118970.

In general, nucleic acid encoding antibody heavy and light chain sequences can be isolated from natural sources and/or obtained by synthetic or semi-synthetic methods. For example, the hybridoma cells produced as described above can serve as a suitable source of DNA for recombinant antibody production.

Nucleic acid encoding surrogate light chain, e.g. VpreB and λ5 polypeptides, can be isolated from natural sources, e.g. developing B cells and/or obtained by synthetic or semi-synthetic methods. Once this DNA has been identified and isolated or otherwise produced, it can be ligated into a replicable vector for further cloning or for expression.

Similarly, nucleic acid encoding the chimeric fusion polypeptides of the present invention can be produced by synthetic or semi-synthetic means, and ligated into a replicable vector for cloning or expression.

In the methods of the present disclosure, typically antibody light chains and antibody heavy chains are at first cloned separately. Because the sequences present in the vectors harbor the coding sequences of the antibody heavy and light chains separately, the sequences may be excised and inserted into one or more expression vectors for expression of the antibody heavy and light chains. Preferably, the coding sequences of the antibody heavy and light chains, are inserted into the same expression vector for coexpression of the heavy and light chains to produce antibody libraries. The same approach can be used to produce antibody-based chimeric fusion polypeptides.

Surrobody™ sequences, including Surrobody™ -based chimeric fusion molecules, may be cloned in one or multiple vectors, depending on the structure (e.g. dimeric or trimeric) in question.

The expression vectors of suitable for the expression of antibody chains (including antibody-based chimeric fusion proteins) or components of the Surrobody™ constructs (including Surrobody™ -based chimeric fusion proteins) contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Examples of suitable mammalian host cell lines include, without limitation, monkey kidney CV1 line transformed bySV40 (COS-7, ATCC CRL 1651); human embryonic kidney line 293 (293 cells) subcloned for growth in suspension culture, Graham et al, J. Gen Virol. 36:59 (1977)); baby hamster kidney cells (BHK, ATCC CCL 10); Chinese hamster ovary cells/- DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; mouse myeloma NS0 cells; and a human hepatoma line (Hep G2).

For use in mammalian cells, the control functions on the expression vectors are often provided by viral material. Thus, commonly used promoters can be derived from the genomes of polyoma, Adenovirus2, retroviruses, cytomegalovirus, and Simian Virus 40 (SV40). Other promoters, such as the β-actin protomer, originate from heterologous sources. Examples of suitable promoters include, without limitation, the early and late promoters of SV40 virus (Fiers et al., Nature, 273: 113 (1978)), the immediate early promoter of the human cytomegalovirus *(*Greenaway et al., Gene, 18: 355-360 (1982)), and promoter and/or control sequences normally associated with the desired gene sequence, provided such control sequences are compatible with the host cell system.

Transcription of a DNA encoding a desired heterologous polypeptide by higher eukaryotes is increased by inserting an enhancer sequence into the vector. The enhancer is a cis-acting element of DNA, usually about from 10 to 300 bp, that acts on a promoter to enhance its transcription-initiation activity. Enhancers are relatively orientation and position independent, but preferably are located upstream of the promoter sequence present in the expression vector. The enhancer might originate from the same source as the promoter, such as, for example, from a eukaryotic cell virus, e.g. the SV40 enhancer on the late side of the replication origin (bp 100-270), the cytomegalovirus early promoter enhancer, the polyoma enhancer on the late side of the replication origin, and adenovirus enhancers.

Expression vectors used in mammalian host cells also contain polyadenylation sites, such as those derived from viruses such as, e.g., the SV40 (early and late) or HBV.

An origin of replication may be provided either by construction of the vector to include an exogenous origin, such as may be derived from SV40 or other viral (e.g., Polyoma, Adeno, VSV, BPV) source, or may be provided by the host cell.
Expression vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the antibodies-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the antibody-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding antibodies

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

Transcription of the heavy chain or light chain genes or nucleic acid encoding Surrobody™ chains or components in the expression vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the antibody genes, Surrobody sequences, or the chimeric fusion polypeptides of the present invention by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the antibody coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding antibody heavy and light chains.

Still other methods, vectors, and host cells suitable for adaptation to the synthesis of polypeptide, in recombinant vertebrate cell culture are described in Gething et al., Nature, 293:620-625 (1981); Mantei et al., Nature, 281:40-46 (1979); EP 117,060; and EP 117,058.

The coding sequences of the individual chains within a multi-chain construct comprising functionally null surrogate light chain constructs, including the chimeric fusion proteins herein, can be present in the same expression vector, under control of separate regulatory sequences, or in separate expression vectors, used to cotransfect a desired host cells, including eukaryotic and prokaryotic hosts. Thus, multiple genes can be coexpressed using the Duet™ vectors commercially available from Novagen.

The transformed host cells may be cultured in a variety of media. Commercially available media for culturing mammalian host cells include Ham's F10 (Sigma), Minimal Essential Medium ((MEM), (Sigma), RPMI-1640 (Sigma), and Dulbecco's Modified Eagle's Medium ((DMEM), Sigma). In addition, any of the media described in Ham et al., Meth. Enz. 58:44 (1979) and Barnes et al., Anal. Biochem. 102:255 (1980) may be used as culture media for the host cells. The culture conditions, such as temperature, pH, and the like, are those previously used with the host cell selected for expression, and are included in the manufacturer's instructions or will otherwise be apparent to the ordinarily skilled artisan.

Further suitable media for culturing mammalian, bacterial (e.g. *E. coli*) or other host cells are also described in standard textbooks, such as, for example, Sambrook et al., *supra,* or Ausubel et al., *supra.*

Purification can be performed by methods known in the art. In a preferred embodiment, the surrogate antibody molecules are purified in a 6xHis-tagged form, using the Ni-NTA purification system (Invitrogen).

Domain antibodies (dABs) are the smallest functional binding units of antibodies, corresponding to the variable regions of either the heavy (VH) or light (VL) chains of human antibodies. dABs have a molecular weight of approximately 13 kDa, or less than one-tenth the size of a full antibody.
dAbs are well expressed in bacterial, yeast, and mammalian cell systems, and can be produced in a manner similar to the recombinant production of antibodies, following methods described above and in the rest of the specification.

Similarly, Adnectins, a class of targeted biologics, which consist of the natural fibronectin backbone, as well as the multiple targeting domains of a specific portion of human fibronectin, can be produced by well known recombinant DNA techniques, such as those discussed above and in the rest of the specification.

### Preparation of surrogate light chain constructs

Cloning and expression vectors that can be used for expressing the coding sequences of the polypeptides herein are well known in the art and are commercially available. The vector components generally include, but are not limited to, one or more of the following: a signal sequence, an origin of replication, one or more marker genes, an enhancer element, a promoter, and a transcription termination sequence. Suitable host cells for cloning or expressing the DNA encoding the surrogate light chain constructs in the vectors herein are prokaryote, yeast, or higher eukaryote (mammalian) cells, mammalian cells are being preferred.

Examples of suitable mammalian host cell lines include, without limitation, monkey kidney CV1 line transformed bySV40 (COS-7, ATCC CRL 1651); human embryonic kidney line 293 (293 cells) subcloned for growth in suspension culture, Graham et al, J. Gen Virol. 36:59 (1977)); baby hamster kidney cells (BHK, ATCC CCL 10); Chinese hamster ovary cells/- DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

For use in mammalian cells, the control functions on the expression vectors are often provided by viral material. Thus, commonly used promoters can be derived from the genomes of polyoma, Adenovirus2, retroviruses, cytomegalovirus, and Simian Virus 40 (SV40). Other promoters, such as the β-actin protomer, originate from heterologous sources. Examples of suitable promoters include, without limitation, the early and late promoters of SV40 virus (Fiers et al., Nature, 273: 113 (1978)), the immediate early promoter of the human cytomegalovirus *(*Greenaway et al., Gene, 18: 355-360 (1982)), and promoter and/or control sequences normally associated with the desired gene sequence, provided such control sequences are compatible with the host cell system.

Transcription of a DNA encoding a desired heterologous polypeptide by higher eukaryotes is increased by inserting an enhancer sequence into the vector. The enhancer is a cis-acting element of DNA, usually about from 10 to 300 bp, that acts on a promoter to enhance its transcription-initiation activity. Enhancers are relatively orientation and position independent, but preferably are located upstream of the promoter sequence present in the expression vector. The enhancer might originate from the same source as the promoter, such as, for example, from a eukaryotic cell virus, e.g. the SV40 enhancer on the late side of the replication origin (bp 100-270), the cytomegalovirus early promoter enhancer, the polyoma enhancer on the late side of the replication origin, and adenovirus enhancers.

Expression vectors used in mammalian host cells also contain polyadenylation sites, such as those derived from viruses such as, e.g., the SV40 (early and late) or HBV.

An origin of replication may be provided either by construction of the vector to include an exogenous origin, such as may be derived from SV40 or other viral (e.g., Polyoma, Adeno, VSV, BPV) source, or may be provided by the host cell.

The expression vectors usually contain a selectable marker that encodes a protein necessary for the survival or growth of a host cell transformed with the vector. Examples of suitable selectable markers for mammalian cells include dihydrofolate reductase (DHFR), thymidine kinase (TK), and neomycin.

Suitable mammalian expression vectors are well known in the art and commercially available. Thus, for example, the surrogate light chain constructs of the present invention can be produced in mammalian host cells using a pCI expression vector (Promega), carrying the human cytomegalovirus (CMV) immediate-early enhancer/promoter region to promote constitutive expression of a DNA insert. The vector can contain a neomycin phosphotransferase gene as a selectable marker.

The surrogate light chain constructs of the present invention can also be produced in bacterial host cells. Control elements for use in bacterial systems include promoters, optionally containing operator sequences, and ribosome binding sites. Suitable promoters include, without limitation, galactose (gal), lactose (lac), maltose, tryptophan (trp), β-lactamase promoters, bacteriophage λ and T7 promoters. In addition, synthetic promoters can be used, such as the tac promoter. Promoters for use in bacterial systems also generally contain a Shine-Dalgarno (SD) sequence operably linked to the DNA encoding the Fab molecule. The origin of replication from the plasmid pBR322 is suitable for most Gram-negative bacteria.

The coding sequences of the individual chains within a multi-chain construct comprising antibody surrogate light chain sequences can be present in the same expression vector, under control of separate regulatory sequences, or in separate expression vectors, used to cotransfect a desired host cells, including eukaryotic and prokaryotic hosts. Thus, multiple genes can be coexpressed using the Duet™ vectors commercially available from Novagen.

The transformed host cells may be cultured in a variety of media. Commercially available media for culturing mammalian host cells include Ham's F10 (Sigma), Minimal Essential Medium ((MEM), (Sigma), RPMI-1640 (Sigma), and Dulbecco's Modified Eagle's Medium ((DMEM), Sigma). In addition, any of the media described in Ham et al., Meth. Enz. 58:44 (1979) and Barnes et al., Anal. Biochem. 102:255 (1980) may be used as culture media for the host cells. The culture conditions, such as temperature, pH, and the like, are those previously used with the host cell selected for expression, and are included in the manufacturer's instructions or will otherwise be apparent to the ordinarily skilled artisan.

Further suitable media for culturing mammalian, bacterial (e.g. *E. coli*) or other host cells are also described in standard textbooks, such as, for example, Sambrook et al., *supra,* or Ausubel et al., *supra.*

Purification can be performed by methods known in the art. In a preferred embodiment, the surrogate antibody molecules are purified in a 6xHis-tagged form, using the Ni-NTA purification system (Invitrogen).

### Examples of functionally null antibodies and Surrobodies

An antibody or Surrobody is functionally null if it is does not appreciably bind a target under conditions of its use. A functionally null phenotype can be accomplished through several means. One such functionally null agent is one that specifically targets inaccessible targets, like foreign antigens not present in the host organism, or physically inaccessible intracellular or nuclear antigens. *Bona fide* anti-pathogen antibodies are immune tolerant because they do not measurably recognize accessible self-antigens in an individual and by definition are "functionally null" in the absence of a circulating target. Such antibodies can be isolated and propagated directly, by a variety of techniques known in the art, for the identification of anti-pathogen antibodies.

Another example of a functionally null agent is one with binding specificity towards a target that is present at such limited quantities that, if sequestered, it would not appreciably deplete the functionally null reagent. This can be accomplished by screening for binders against targets that are very rate,

Another example of a scaffold with functionally null binding region(s) is one that binds with unfavorable equilibrium kinetics, whereby the affinity for the target is disproportionately distanced from the target abundance such that the agent is not appreciably sequestered. In this instance a screening is performed that produces specific but very weak binders to the target.

Yet another example of a scaffold with a functionally null binding region is a preliganded agent whose binding space is occupied with target, to render it null and nonreactive. This can be achieved, for example, by stably combining the specific target with the specific binding agent into a non-reactive complex.

Another approach for generating a scaffold with a non-reactive functionally null binding region is to recombinantly express the scaffold, or fragment thereof, along with the binder to render it nonreactive.

A further example of a scaffold according to the present disclosure is a moiety that serendipitously does not bind to any homogeneous or heterogeneous targets.

### Design of functionally null antibodies or Surrobodies

Recombinantly null antibodies can be generated through several strategies. One strategy is to create germline heavy and light chains composed of unmutated V-J light chains and unmutated V-D-J heavy chains to encode null antibody polypeptides. As most binding by an antibody is dictated by the heavy chain CDR3, another option to further reduce the likelihood of binding to any target is to remove or design a minimal nonreactive D-region. It may be further possible and necessary to remove not only the D-region but to also truncate portions of the C-terminal V region and truncate the N-terminal J region in order to engineer a null heavy chain. Specifically the deletions one may consider would be to shorten the V-region up to Kabat residue 90 and making a similar truncation of the the J region up to Kabat residue 106. Should the resulting unmutated V-D-J recombined polypeptides or aforementioned deletion strategies have unfavorable binding characteristics or unfavorable structural characteristics one can then engineer site directed substitutions at any or all residues between V-region Kabat residue 90 and J-region Kabat region 106. The substitutions one would consider would likely first be amino acids with minimally chemically reactive side chains such as glycine or alanine, but substitutions would not be restricted to these amino acids. All of the methods described above to generate nonreactive heavy chains are similarly useful in the design of functionally null Surrobodies.

### Identifying functionally null molecules from diverse displayed collections

Large collections of diversified Surrobody, antibody, or other CDR-containing immunoglobulin-related libraries can be created by using rescued lymphocyte-based diversity or through entirely chemical, synthetic means. These collections are well known in the art and are typically used to identify target specific binders through iterative methods of positive selection. Frequently subtractive steps are used to remove nonspecific binders prior to, or during the positive enrichment. To date, every known screen is always concluded with a target specific positive selection followed by discarding the non-binders. To identify functionally null candidates a single subtractive screen can be utilized to categorically identify non-binders. To reinforce the non-binding status of a collection of clones, one can engage an iterative subtractive screen, with a single background or a combination of relevant backgrounds where non-binding is desirable.

In practice, to find agents that do not bind within the systemic vasculature or to circulating cells, it is possible to intravenously inject a diversified library into a rat and after an appropriate amount of time recover library members directly from the serum of the test subject. Repeating the process with a rat, or other tractable animal, can reinforce the enrichment of a nonbinding collection. Suitable process controls can be developed and incorporated to eliminate any unwanted serum component binders by approaches similar to those commonly utilized and described to eliminate undesirable binders from screens.

In another example, these steps can be recapitulated *in vitro* with vascular tissue or vascular cell-based negative selection, as well as whole blood negative selection to identify possible circulating nonbinders. This *in vitro* approach is particularly useful in cases of intractable species, such as human subjects, where for practical and ethical purposes one would be precluded from performing the previously described *in vivo* selection steps.

In another instance, one the *in vivo* selection in animals can be combined with the *in vitro* steps using isolated agents to gain greatest confidence that nonbinding agents have been selected.

Identification of the best functionally null candidates can be achieved by multiparameter binding assays confirming nonreactivity to the selected backgrounds and good production of the functionally null scaffold. In each of these instances, a library can be displayed by any of the commonly used methods of display, such as, for example, the display methods described below.

While the various techniques have been discussed with reference to antibodies and antibody-like molecules, it is also be possible to extend these types of screens to identify non-binding collections and clones from libraries of non-immunoglobulin-related scaffolds, such as, for example, Adnectins, DARPins, anti-calins, and Affibodies.

### Collections of functionally null antibodies, Surrobodies, dAbs, Adnectins, and Libraries comprising the functionally null antibodies, Surrobodies or chimeric constructs herein

Collections of functionally null antibodies, Surrobodies, dAbs, Adnectins, and similar molecules, or chimeric fusion molecules herein can be present in libraries, which are preferably in the form of a display.

Systems for displaying heterologous proteins, including antibodies, Surrobodies, and other polypeptides, are well known in the art. Antibody fragments have been displayed on the surface of filamentous phage that encode the antibody genes (Hoogenboom and Winter J. Mol. Biol., 222:381 388 (1992); McCafferty et al., Nature 348(6301):552 554 (1990); Griffiths et al. EMBO J., 13(14):3245-3260 (1994)). For a review of techniques for selecting and screening antibody libraries see, *e.g.,* Hoogenboom, Nature Biotechnol. 23(9):1105-1116 (2005). In addition, there are systems known in the art for display of heterologous proteins and fragments thereof on the surface of *Escherichia coli* (Agterberg et al., Gene 88:37-45 (1990); Charbit et al., Gene 70:181-189 (1988); Francisco et al., Proc. Natl. Acad. Sci. USA 89:2713-2717 (1992)), and yeast, such as *Saccharomyces cerevisiae* (Boder and Wittrup, Nat. Biotechnol. 15:553-557 (1997); Kieke et al., Protein Eng. 10:1303-1310 (1997)). Other known display techniques include ribosome or mRNA display (Mattheakis et al., Proc. Natl. Acad. Sci. USA 91:9022-9026 (1994); Hanes and Pluckthun, Proc. Natl. Acad. Sci. USA 94:4937-4942 (1997)), DNA display (Yonezawa et al., Nucl. Acid Res. 31(19):e118 (2003)); microbial cell display, such as bacterial display (Georgiou et al., Nature Biotech. 15:29-34 (1997)), display on mammalian cells, spore display (Isticato et al., J. Bacteriol. 183:6294-6301 (2001); Cheng et al., Appl. Environ. Microbiol. 71:3337-3341 (2005) and co-pending provisional application Serial No. 60/865,574, filed November 13, 2006), viral display, such as retroviral display (Urban et al., Nucleic Acids Res. 33:e35 (2005), display based on protein-DNA linkage (Odegrip et al., Proc. Acad. Natl. Sci. USA 101:2806-2810 (2004); Reiersen et al., Nucleic Acids Res. 33:e10 (2005)), and microbead display (Sepp et al., FEBS Lett. 532:455-458 (2002)).

For the purpose of the present disclosure, the functionally null antibodies, Surrobodies, and other scaffolds comprising one or more functionally null binding regions, can be displayed by any display technique, such are, for example, phage display, yeast display, or spore display.

In phage display, the heterologous protein, such as a Surrobody or an antibody sequence, is linked to a coat protein of a phage particle, while the DNA sequence from which it was expressed is packaged within the phage coat. Details of the phage display methods can be found, for example, McCafferty et al., Nature 348, 552-553 (1990)), describing the production of human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell.

Phage display can be performed in a variety of formats; for their review see, e.g. Johnson, Kevin S. and Chiswell, David J., Current Opinion in Structural Biology 3, 564-571 (1993). Several sources of heavy chain V-gene segments can be discovered through phage display. Clarkson et al., Nature 352, 624-628 (1991) isolated a diverse array of anti-oxazolone heavy chains and light chains from a small random combinatorial library of V genes derived from the spleens of immunized mice. A repertoire of heavy and light chain V genes from unimmunized human donors can be constructed and recovered specific to a diverse array of antigens (including self-antigens) essentially following the techniques described by Marks et al., J. Mol. Biol. 222, 581-597 (1991), or Griffith et al., EMBO J. 12, 725-734 (1993). These, and other techniques known in the art, can be adapted to the display of any polypeptide, including polypeptides and other constructs comprising surrogate light chain sequences. Thus, for example, the surrogate light chain can be supplemented with a collection of heavy chains from either a naturally diverse source, such as lymphocytes, or a synthetically generated collection created entirely through techniques of molecular biology. These collections can be cloned, expressed and selected, by methods known in the art.

Spore display systems are based on attaching the sequences to be displayed to a coat protein, such as a *Bacillus subtilis* spore coat protein. The spore protoplast (core) is surrounded by the cell wall, the cortex, and the spore coat. Depending on the species, an exosporium may also be present. The core wall is composed of the same type of peptidoglycan as the vegetative cell wall. Spore display, including surface display system using a component of the *Bacillus subtilis* spore coat (CorB) and *Bacillus thuringiensis (Bt)* spore display, is described in Isticato et al., J. Bacteriol. 183:6294-6301 (2001); Cheng et al., Appl. Environ. Microbiol. 71:3337-3341 (2005), the entire disclosures of which is hereby expressly incorporated by reference. Various spore display techniques are also disclosed in U.S. Patent Application Publication Nos. 20020150594; 20030165538; 20040180348; 20040171065; and 20040254364, the entire disclosures are hereby expressly incorporated by reference herein.

An advantage of spore display systems is the homogenous particle surface and particle size of non-eukaryotic nature, which is expected to provide an ideal non-reactive background. In addition, the particle size of spores is sufficient to enable selection by flow cytometry that permits selectable clonal isolation, based upon interactions.

Leveraging on the stability of spores, it is possible to perform various post-sporulation chemical, enzymatic and/or environmental treatments and modification. Thus, it is possible to stabilize structural helical structures with chemical treatment using trifluoroethanol (TFE), when such structures are displayed. In addition, oxidative stress treatments, such as treatments with Reactive Oxygen Species (e.g. peroxide) or reactive Nitrogen Species (e.g. nitrous acid) are possible. It is also possible to expose defined or crude populations of spore-displayed polypeptides to enzymatic treatments, such as proteolytic exposure, other enzymatic processes, phosphorylation, etc. Other possible treatments include, without limitation, nitrosylation by peroxynitrite treatment, proteolysis by recombinant, purified, or serum protease treatment, irradiation, coincubation with known chaperones, such as heat shock proteins (both bacterial and mammalian), treatment with folding proteins, such as protein disulfide isomerase, prolyl isomerase, etc., lyophilization, and preservative-like treatments, such as treatment with thimerosol. These treatments can be performed by methods well known in the art.

Similar techniques can be used in all spore display systems, including displays where the attachment is to a spore coat protein, including, for example, the spore display systems disclosed in U.S. Publication No. 20090098164, published April 16, 2009.

### Uses of the conjugates herein

As discussed earlier, the approach of the present invention can be used to improve one or more pharmacokinetic properties of a moiety, such as a protein or peptide, by conjugation, e.g. recombinant fusion, to a non-binding scaffold that can confer the desired improvement on the moiety to which it is conjugated (fused). Thus, for example, the half-lives of biologically active moieties can be modulated (extended or shortened) by conjugation to a long or short-lived functionally null moiety. If the half-life is extended, the consequence is improvement of the efficacy by either reducing doses or the frequency of dosing for particular disorders. Because it uses intact antibody or Surrobody technologies one can expect better tolerance and pharmacokinetic properties compared to Fc fusions, as well as improved manufacturability.

Specific examples of peptide or polypeptide drugs, which can benefit from the approach of the present invention include, without limitation are interferon alpha (IFN-α), interferon beta (IFN-β), calcitonin, parathyroid hormone, BAFF-r, TACI, FSH, Interleukin-2, erythropoietin, G-CSF, GM-CSF, Factor VII, DNAse, hirudin, urokinase, streptokinasae, Growth hormone, Glucagon, Kremen-1, Kremen-2, HGF, FGF-21, GLP-1, Exendin-4, Oxyntomodulin, amylin, PACAP, T20 HIV inhibitory peptide, IL-22, Thrombospondin peptide fragments, BMP-7, CTLA-IV, t-PA, Flt-1, IL-1ra, Insulin, melanocortin, herstatin, amylin, conotoxins, TNF-RI, GITR, and Gastrin. Also all variants of the preceding list of proteins are expected to benefit from this approach. Other examples of agents that would benefit from Surrobody association are the Epo agonist and Tpo agonist peptide mimetics.

Further examples which can benefit from this approach of the present invention include, without limitation are antibody fragments, scFv, nanobodies, and similar derivatives.

Protein scaffolds that can benefit from this approach include, also without limitation are avimers, phylomers, DARPins, anti-calins, adnectins, tetranectins, and other binding proteins reprogrammed to engender novel or enhanced activities.

Additional examples which can benefit from this approach are peptides and polypeptides known to associate specifically to non-peptidic haptens. For this type of Surrobody the fused element extends the half-life of associated bound element that may be the active pharmaceutical or a conjugated element to an active pharmaceutical. An example of this is an FKBP12 Surrobody that binds and extends the half-life of the FK-506 macrolide, or to an active pharmaceutical molecule fused to FK-506. Alternatively rapamycin can be similarly used in conjunction with the FKBP12 Surrobody. Other naturally occurring or designed specific peptide-binder combinations can be utilized to similar effect. Examples of this are leucine zipper peptides, SH2 domains with phosphopeptides, ATP binding domains and ATP analogs, and proteases and either irreversible inhibitors or slowly dissociating inhibitors.

In another aspect, the disclosure provides a conjugate or fusion molecule described herein for use in the preparation or manufacture of a medicament. The medicament may be used to modulate a pharmacokinetic property *in vivo* as described herein. The medicament may also be used for the treatment of a condition or disorder described herein. In one embodiment, the medicament may be used to reduce plasma glucose levels in a subject in need.

In another aspect, the disclosure provides a conjugate or fusion molecule described herein for use in a method of reducing plasma glucose levels in a subject in need. In one embodiment, the method includes the step of administering an effective amount of a conjugate comprising (i) a first moiety comprising a biologically active GLP-1 receptor agonist, and (ii) a second moiety, wherein the second moiety is a scaffold comprising one or more functionally null binding regions conjugated to and capable of modulating at least one pharmacokinetic property of the first moiety. In another embodiment, the method includes the step of administering an effective amount of a fusion molecule comprising (i) a first moiety comprising a biologically active GLP-1 receptor agonist, and (ii) a second moiety comprises one or more functionally null binding regions fused to and capable of modulating at least one pharmacokinetic property of the first moiety. The reduction in glucose levels may be an acute and/or a prolonged reduction of glucose levels. As demonstrated in Example 4, one hour after administration of a GLP-1 Surrobody test animals demonstrated a considerable reduction in glucose levels, which was maintained even at 4 and 8 hours following administration. By contrast, the GLP-1 peptide control was unable to maintain the same degree of blood glucose levels at 4 and 8 hours after administration.

In general, the GLP-1 receptor agonists are biologically active ligands for the GLP-1 receptor and bind the receptor activating it to induce a biological response, or to enhance the preexisting biological activity of the receptor. The biological responses/activities of the GLP-1 receptor include, without limitation, one or more of the following: stimulation of the adenylyl cyclase pathway, increased insulin synthesis, and release of insulin. The biological activity of the GLP-1 receptor agonists include, without limitation, increasing insulin secretion from the pancreas in a glucose-dependent manner; decreasing glucagon secretion from the pancreas by engagement of a G-Protein coupled receptor; increasing insulin-sensitivity in both alpha cells and beta cells; increasing beta cells mass and insulin gene expression, post-translational processing and incretion; inhibiting acid secretion and gastric emptying in the stomach; decreasing food intake by increasing satiety; and promoting insulin sensitivity.

In some embodiments, the GLP-1 receptor agonist may be a peptide or polypeptide. The peptide or polypeptide agonists include, without limitation, GLP-1, Exendin-4/exenatide (Amylin), liraglutide (Novo-Nordisk), albiglutide (GlaxoSmithKline), taspoglutide (Roche), AVE0010/lixisenatide (Sanofi-Aventis), CJC11310PC (Conjuchem), CJC-1131, and variants or fragments thereof. Those of ordinary skill in the art will appreciate other suitable GLP-1 receptor agonists.

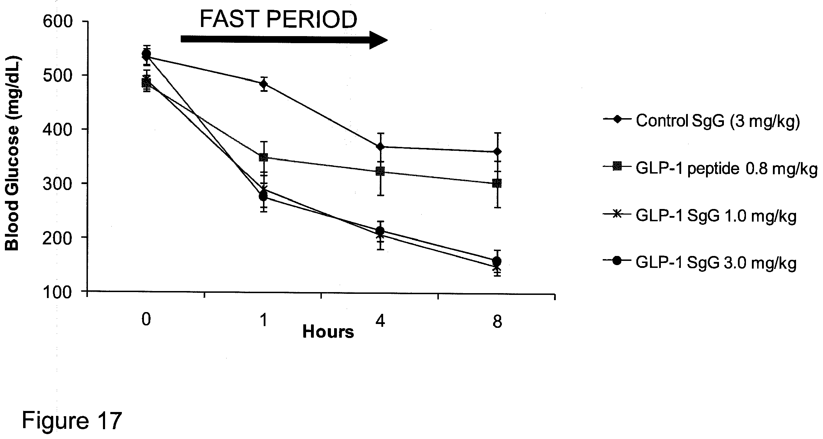

In some embodiments, the pharmacokinetic property modulated is one or more of the following: *in vivo* half-life, clearance, rate of elimination, volume of distribution, degree of tissue targeting, and degree of cell type targeting of GLP-1 receptor agonists. In one embodiment, the *in vivo* half-life of GLP-1 receptor agonists is modulated. In a preferred embodiment, the *in vivo* half-life of a GLP-1 receptor agonist is increased. In one other embodiment, the increase in GLP-1 receptor agonist half-life observed for a conjugate or fusion molecule comprising a first moiety with a GLP-1 receptor agonist and a second moiety with one or more functionally null binding regions (as described herein), is an increase relative to a GLP-1 receptor agonist that lacks the second moiety. For instance, Example 4 and Figure 17 demonstrate that a GLP-1 conjugated functionally null Surrobodies (with a second moiety) displayed a prolonged ability to reduce glucose levels relative to the GLP-1 peptide alone (lacking a second moiety).

In another aspect, the disclosure provides a conjugate or fusion molecule as described herein for use in a method of treating a condition in a subject in need. In one embodiment, the condition is an insulin-related condition. Such conditions include, without limitation, hyperglycemia, low glucose tolerance, insulin resistance, insulin sensitivity, obesity, lipid disorders, dyslipidemia, hyperlipidemia, hypertriglyceridemia, hypercholesterolemia, low HDL levels, high LDL levels, atherosclerosis and its sequelae, vascular restenosis, pancreatitis, abdominal obesity, neurodegenerative disease, retinopathy, nephropathy, neuropathy, and Syndrome X. Those of ordinary skill in the art will appreciate other suitable conditions (see Olson et al. U.S. Patent No. 6,730,690 and O'Neil, et al. U.S. Patent No. 7,833,531 ). In one embodiment, the method includes the step of administering an effective amount of a conjugate comprising (i) a first moiety comprising a biologically active GLP-1 receptor agonist, and (ii) a second moiety, wherein the second moiety is a scaffold comprising one or more functionally null binding regions conjugated to and capable of modulating at least one pharmacokinetic property of the first moiety. In another embodiment, the level of blood glucose in the subject is lowered and/or the secretion of insulin from insulin producing cells is increased, thereby treating the condition.

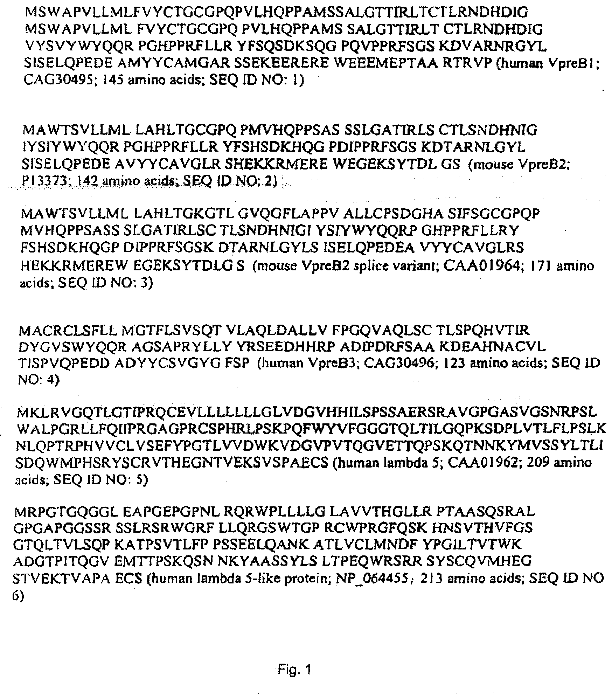

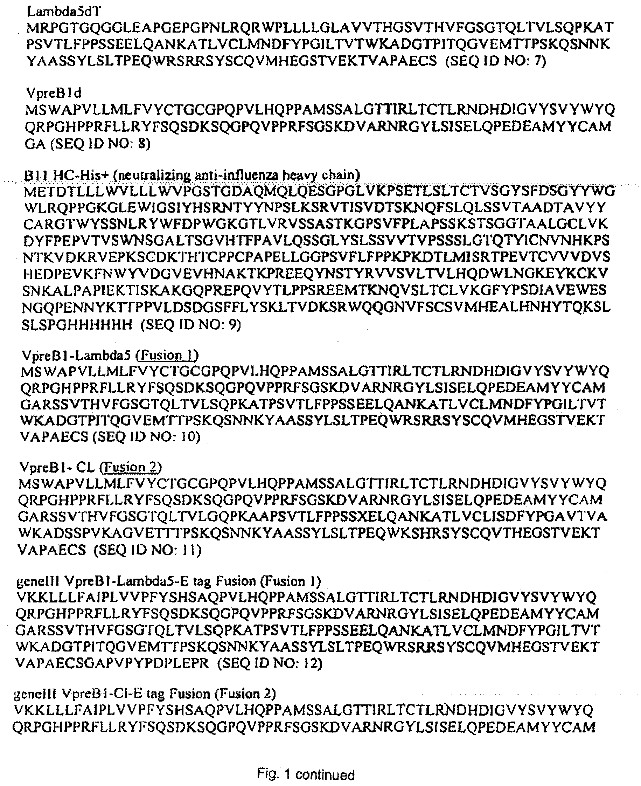

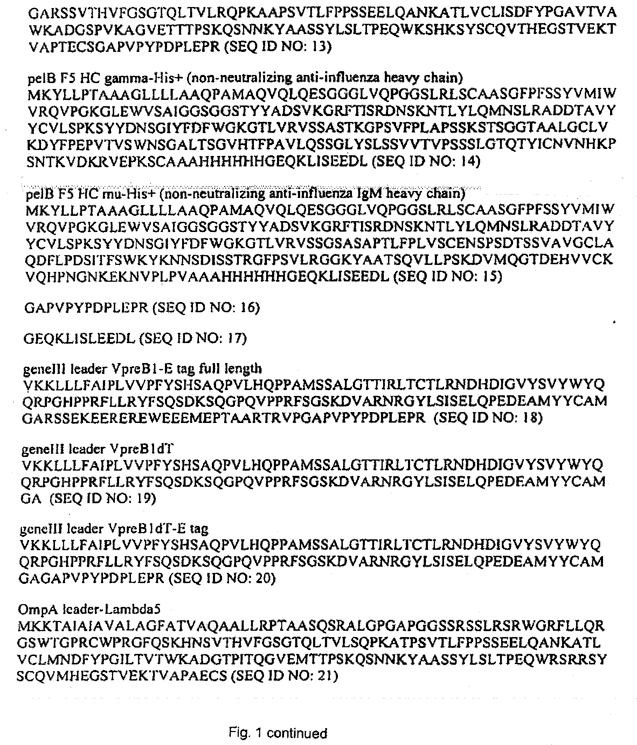

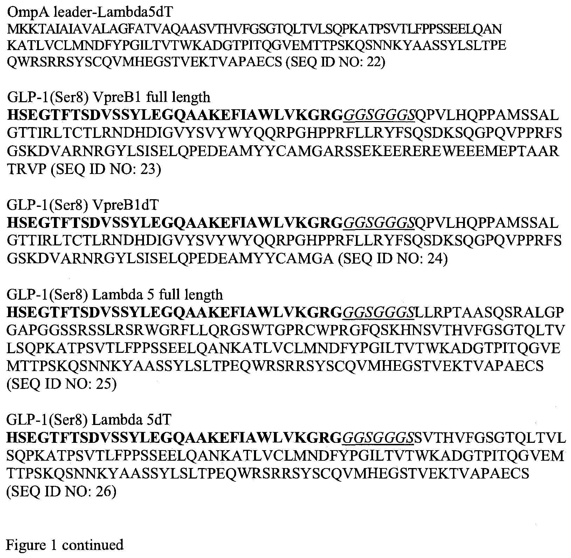

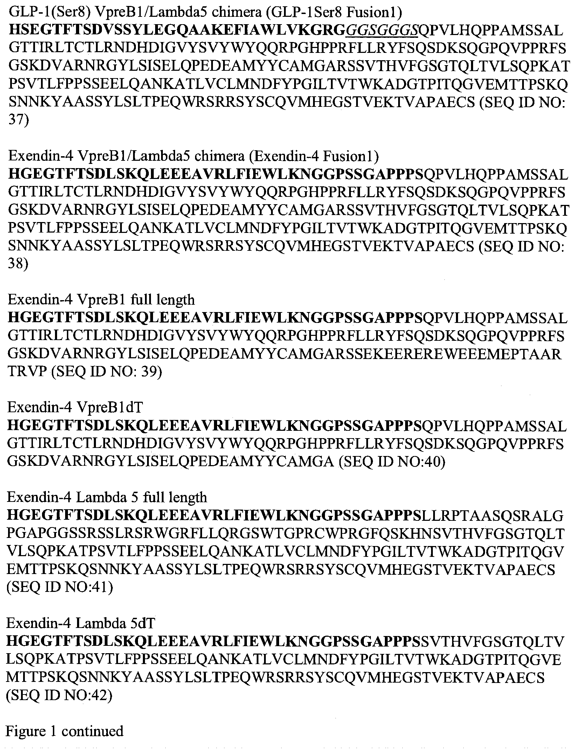

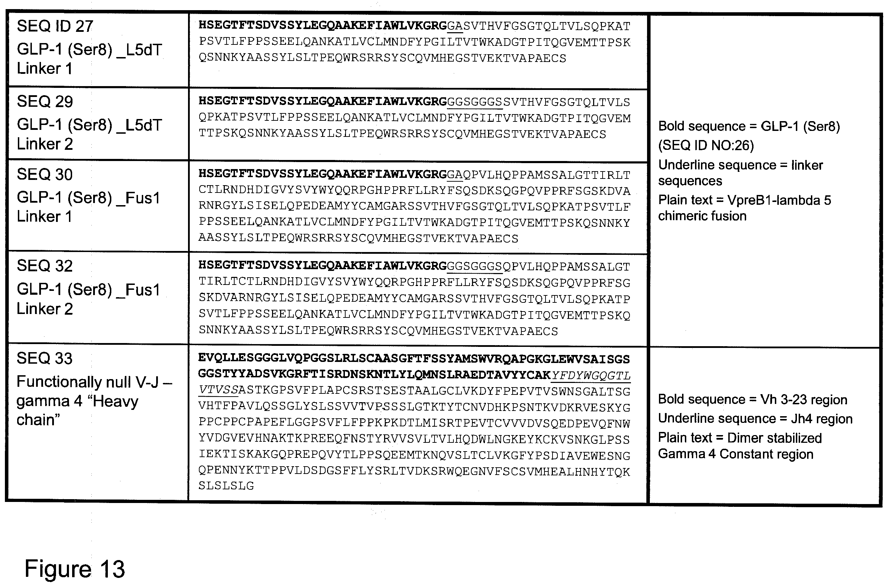

In some embodiments, the present disclosure provides a conjugate or fusion molecule that includes a first moiety with a GLP-1 peptide or polypeptide comprising an amino acid sequence selected from SEQ ID NO: 26, SEQ ID NO:34, or a fragment a variant thereof. Figure 13 contains examples of mature GLP-1 Ser8 SLC sequences. In some embodiments, the present disclosure provides a conjugate or fusion molecule that includes a first moiety with an Exendin-4 peptide or polypeptide comprising an amino acid sequence selected from SEQ ID NO: 35, SEQ ID NO:36, or a fragment a variant thereof. Figure 1 contains examples of Exendin-4 (M→L) SLC sequences.

In another aspect, the present disclosure provides methods of modulating at least one pharmacokinetic property of a molecule. In one embodiment, the method includes the step of conjugating the molecule to a moiety comprising at least one functionally null binding region. The conjugated molecule may be a "conjugate" or "fusion molecule" described herein. In another embodiment, the method includes the step of administering an effective amount of the conjugate or fusion molecule to a subject in need. In one other embodiment, the conjugated molecule has at least one modulated pharmacokinetic property upon administration to the subject as compared to a molecule lacking the at least one functionally null binding region. In yet another embodiment, the at least one pharmacokinetic property of the molecule upon administration to the subject is modulated as compared to the molecule lacking the at least one functionally null binding region. In one embodiment, the at least one modulated pharmacokinetic property is *in vivo* half-life.

### Chimeric Surrobody structures

From the naturally occurring or engineered functionally null antibodies one can incorporate any number and any combinations of the functional components to any of the naturally occurring, or engineered, termini of either the heavy chains and/or light chains.

In the case of functionally null Surrobodies, it is possible to incorporate functional components at the amino and/or carboxyl termini of the heavy chains and/or to the amino and/or carboxyl termini of either or both of surrogate light chain subunits. Additional opportunities exist for sites of incorporation within the surrogate light chain depending upon whether the surrogate light chain subunits are produced as separate polypeptides or as an engineered chimeric fusions. In any instance, the resulting termini are available for chimeric incorporation.

Further opportunities for incorporation exist within either subunit. In the case of the VpreB subunit, the regions defined by residues 49-58 and 68-81 form loops analogous to contact rule defined CDR 1 and CDR2 light chain loops, each of which can be used as sites of functional incorporation or substitution. For λ5 opportunities exist to utilize any number of the solvent exposed residues or loops as for functional incorporation or substitution. The most favored positions are those on the λ5 face opposing the heavy chain dimerizing axis. One unique opportunity for chimeric incorporation is within, or proximal to, the VpreB λ5 junction in the chimeric surrogate light chain fusion. The position is, however, less critical than in the case of functional (binding) Surrobodies. The fact that the constructs are non-binding (functionally null) provides for more flexibility.

While in these descriptions reference is made to antibodies and Surrobodies individually, it is emphasized that similar approaches can be used to make hybrid surrogate light chain/antibody light chain combinations or to break apart light chains. Indeed, hybrid surrogate light chain/antibody light chain combinations are readily generated and may have beneficial binding and biophysical stability properties. Also, even though the preceding examples describe full length proteins and relatively small scale engineered deletions, one skilled in the art can realize the process and formats are valid and applicable for fragments, single chain derivatives, domains, and structures based upon repeated Surrobody and/or antibody domains.

For therapeutic applications, the chimeric fusion polypeptides herein are usually used in the form of pharmaceutical compositions. Techniques and formulations generally may be found in Remington's Pharmaceutical Sciences, 18th Edition, Mack Publishing Co. (Easton, Pa. 1990). See also, Wang and Hanson "Parenteral Formulations of Proteins and Peptides: Stability and Stabilizers," Journal of Parenteral Science and Technology, Technical Report No. 10, Supp. 42-2S (1988). Suitable routes of administration include, without limitation, oral (including buccal, sublingual, inhalation), nasal, rectal, vaginal, and topically.

The chimeric fusion molecules herein may be formulated in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

The fusion molecules also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nanoparticles and nanocapsules), or in macroemulsions. Such techniques are disclosed in Remington's Pharmaceutical Sciences, *supra.*

The fusion polypeptides disclosed herein may also be formulated as immunoliposomes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Pat. Nos. 4,485,045 and 4,544,545; and WO97/38731 published Oct. 23, 1997. Liposomes with enhanced circulation time are disclosed in U.S. Pat. No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al. J. Biol. Chem. 257:286-288 (1982) via a disulfide interchange reaction.

For the prevention or treatment of disease, the appropriate dosage of the fusion polypeptides herein will depend on the type of disease to be treated the severity and course of the disease, and whether the polypeptide is administered for preventive or therapeutic purposes. The fusion polypeptide is suitably administered to the patient at one time or over a series of treatments. Depending on the type and severity of the disease, about 1 µ/kg to about 15 mg/kg of the fusion polypeptide is a typical initial candidate dosage for administration to the patient, whether, for example, by one or more separate administrations, or by continuous infusion.

The selection of the appropriate dose is well within the skill of a practicing clinician.

Further details of the invention are provided by the following examples.

### Example 1: Construction and purification of recombinant GLP-1 conjugated null Surrobodies

The incretin peptide GLP-1 is potent activator of the GLP-1 receptor on pancreatic islet cells. In the presence of elevated glucose, GLP-1 receptor stimulation leads to insulin release that in turn causes insulin sensitive cells to absorb glucose. Administration of GLP-1 to type II diabetics brings about beneficial reductions in plasma glucose levels. However, GLP-1 is rapidly inactivated within 2 minutes by Dipeptidyl protease IV (DPP-IV), severely limiting its therapeutic utility. DPP-IV resistant GLP-1 receptor activators have been created that are longer lasting, but still only have half-lives of approximately 30 minutes due in large part to renal elimination of these relatively small molecular weight agents. While these relative short half-life agents are therapeutically beneficial, they require twice daily injections to see improvements in blood glucose management. Conjugating a DPP-IV resistant GLP-1 receptor agonist with a long lived functionally null agent could provide a highly effective and convenient mode of treatment requiring less frequent injections.

Amino acids 7-37 of GLP-1 contain the amino acid sequence HAEGTFTSDVSSYLEGQAAKEFIAWLVKGRG - SEQ ID NO: 34. Our first step to creating such a conjugated hybrid molecule was to select a GLP-1 receptor agonist that contained a serine for alanine substitution at position 8 (H**S**EGTFTSDVSSYLEGQAAKEFIAWLVKGRG - SEQ ID NO: 26). This GLP-1 (Ser8) peptide is markedly resistant to proteolytic inactivation by of DPP-IV. Next we recombinantly conjugated GLP-1 (Ser8) to the amino-terminus of the surrogate light chain fusion, described previously, via two different intervening linker peptides. The first linker peptide added a Gly-Ala two amino acid linker (Whole protein SEQ ID NO: 27), while the second linker added an intervening heptapeptide (Gly-Gly-Ser-Gly-Gly-Gly-Ser) (SEQ ID NO: 28) (whole protein SEQ ID NO: 29). GLP-1 (Ser8) was also similarly fused to the amino terminus of lambda 5 lacking the nonimmunoglobulin-like N-terminal tail. Similarly to the previous recombinant GLP-1 (Ser8) fusions two types of intervening linkers were tested. The first linker peptide added a Gly-Ala two amino acid linker (Whole protein SEQ ID NO: 30), while the second linker added an intervening heptapeptide (Gly-Gly-Ser-Gly-Gly-Gly-Ser) (SEQ ID NO: 31) (whole protein SEQ ID NO: 32). Each GLP-1 surrogate light chain fusion was coexpressed with a γ4-related functionally null heavy chain (SEQ ID NO: 33). The functionally null heavy chain was created by directly combining germline V and J region to a full length Fc. As the heavy chain lacked a D-region it would be predicted to not bind target. The resulting proteins were transiently transfected and expressed using HEK293-6e cells, as follows. Either 0.05 mg of GLP-1 surrogate light chain fusion expression plasmid and 0.05 mg of functionally null heavy chain expression plasmid were mixed, or 0.033 mg of VpreB 1 expression plasmid, 0.033 mg of λ5 expression plasmid, and 0.033 mg of functionally null heavy chain expression plasmid were mixed in 4.8 ml of culture medium. To the DNA: medium mixture 0.2ml of Polyethyleneimine (1mg/ml) was added, mixed, and followed by a 15 minute room temperature incubation. After the incubation was completed the DNA-PEI mixture was combined in final volume of 100 ml media, containing 1.5x10⁸ cells and returned to shake flask incubator. After 16-24 hours the transfected cells were supplemented with 2.5 ml TN1 Tryptone (20% solution). Proteins containing supernatants were harvested by centrifugation and clarified by 0.22 µm filtration after day 6, or when culture viability decreased to 50%. For every 100 ml cleared supernatants we added 28ml of 5M NaCl and 14ml 10X Binding Buffer (GE #28-9030-59). The resulting buffered supernatants were then purified to homogeneity by either FPLC Protein A or Protein G chromatography, which typically produced proteins of >95% purity.

### Example 2: Serum stability of GLP-1 conjugated null Surrobodies

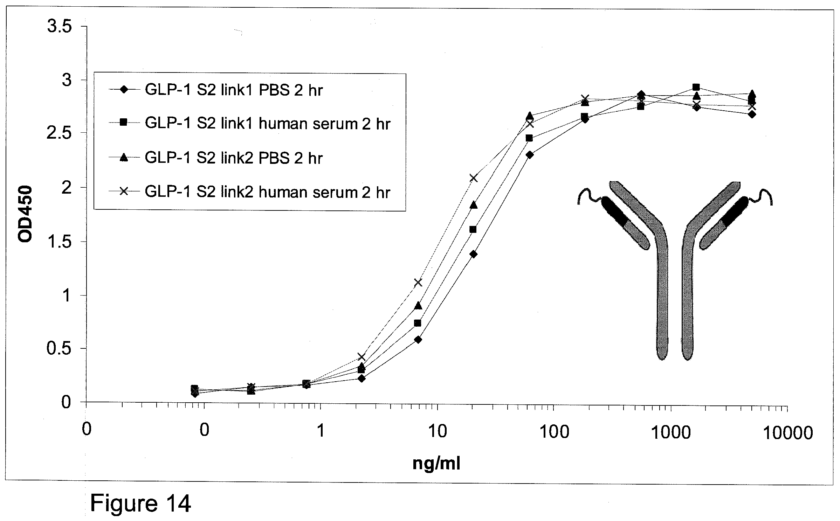

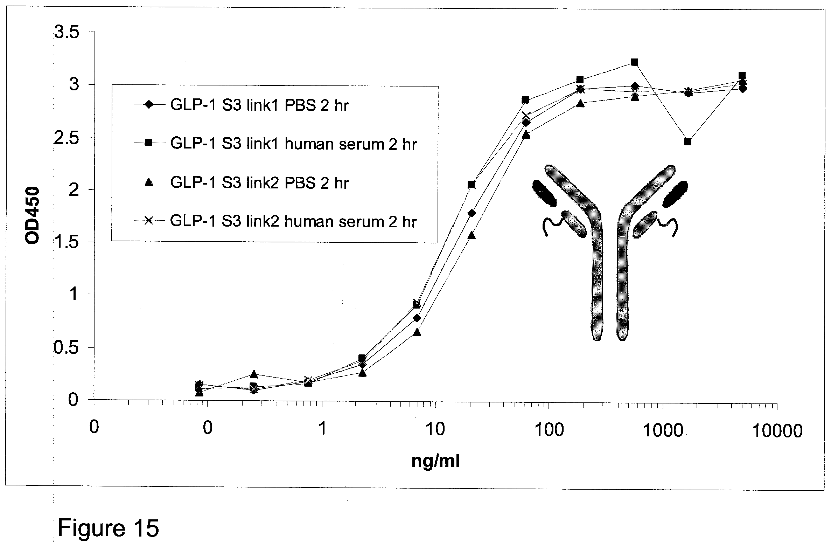

We tested the serum stability of the GLP-1 conjugated null Surrobody following human serum exposure by an ELISA specific to the "active," uncleaved amino terminus of GLP-1. Specifically, we coated microtiter wells with 100 ng of anti-VpreB1 antibody and detected captured GLP-1 Surrobodies through anti-N-terminus (active) GLP-1 antibody. We compared the amount of captured GLP-1-Surrobody following incubation for 2 hours in PBS or human serum at 37°C and found no difference in the quantity of active GLP-1 present (Figure 14: 2 piece Surrobody and Figure 15: 3 piece Surrobody). Figure 14 depicts the serum stability of GLP-1 Two piece S2g Surrobody. Figure 15 depicts the serum stability of GLP-1 Three piece S3g Surrobody. Similar ELISA experiments of GLP-1 SLC paired to heavy chains specific for an antigen utilized an anti-"total" GLP-1 antibody in addition to the anti-active GLP-1 antibody as detection reagents following capture by the anti-VpreB 1 antibody. Additional testing showed that following serum exposure for 3 hours or 10 days there was no measurable decline in total GLP-1 content of the Surrobodies, indicating that linkers were serum stable (data not shown). More importantly, active GLP-1 detection showed no loss after 3 hours of serum incubation and showed very minimal loss after 10 days in serum. In aggregate the results suggest serum resilience of the GLP-1 moiety, the linker, and the surrogate light chain fusion.

### Example 3: Bioactivity of GLP-1 conjugated null Surrobodies

The previous ELISA-based examination of the GLP-1 Surrobodies suggest physiological stability and so we therefore next examined their bioactivity. Common bioactivities for GLP-1 involve typical Gs-linked second messenger systems and reporter assays. GLP-1 receptor, like many GPCRs are subject to β-Arrestin binding that contributes to signal cessation following ligand stimulation. In this reporter assay GLP-1 receptor recombinantly fused to an amino terminal fragment of β-galactosidase (donor), while β-Arrestin is recombinantly fused to an amino terminal deletion mutant of β-galactosidase. When both of these recombinant constructs are present in cells the interaction of β-Arrestin and the GPCR following ligand stimulation forces the complementation of the two β-galactosidase fragments resulting in the formation of a functional enzyme that converts the β-galactosidase chemiluminescent substrate to detectable luminescent signal (DiscoverX - PathHunter Express).

A frozen aliquot of cells expressing GLP-1R/β-galactosidase (donor) and β-Arrestin/β-galactosidase (Acceptor) were seeded in 96-well and allowed to equilibrate for 48 hours at 37°C in 0.1ml OCC media (DiscoverX). Next 0.01 ml of test compounds were added to the cells and then incubated for 90 minutes at 37°C. Following the drug treatment 0.055ml of working detection reagent solution (DiscoverX) was added and then incubated for 90 minutes at room temperature. After this ambient incubation the wells of the resulting plates were read using chemiluminescent plate reader.

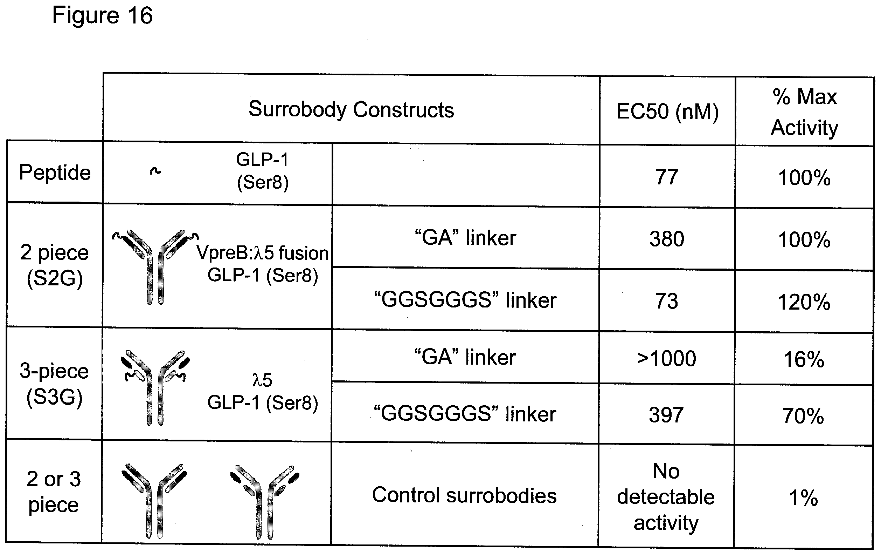

Figure 16 shows a GLP-1 (Ser8) Surrobody with a 7 amino acid "GGSGGGS" linker (SEQ ID NO: 28) had equivalent potency to the GLP-1 Ser8 positive control peptide and had better potency than a similar molecule utilizing a 2 amino acid "GA" linker. Figure 16 shows that GLP-1 Surrobodies activate stable GLP-1 Receptor Reporter Cells.

In this study, GLP-1 recombinant functionally null Surrobodies displayed potencies and efficacies similar to parental synthetic peptides.

### Example 4: Reduction of elevated fasting plasma glucose levels in diabetic mice with GLP-1 conjugated null Surrobodies

Fasting glucose reduction was observed in db/db mice following GLP-1 functionally null Surrobody treatment. Synthetic GLP-1 peptide and recombinant GLP-1 Surrobody fusions were evaluated for their ability to lower plasma glucose in db/db mice. To reduce the effects of in vivo DPPIV proteolysis during the time course of the study GLP-1 both synthetic and recombinant GLP-1 functionally null Surrobodies used a GLP-1 variant containing a serine substitution for alanine at position 8 that reduces DPPIV inactivation. 8 db/db mice were used per group: Control SgG (3 mg/kg) (functionally null SgG that lacks the GLP-1 conjugate); GLP-1 peptide (0.8 mg/kg); GLP-1 SgG (1 mg/kg); and GLP-1 (3 mg/kg). Essentially db/db mice were matched by weight and relative blood glucose levels. Next, the subjects were injected intravenously with test article and isolated with access to water, but not food. Glucose levels were monitored by handheld glucometer after 1 hour and 4 hours. After 4 hours the mice were allowed free access to both food and water. An additional reading was made at 8 hours following drug administration.

Figure 17 shows GLP-1 (1 and 3 mg/kg) acutely reduces blood glucose through 8 hours. In this study GLP-1 Surrobody treated mice showed considerable blood glucose reductions after 1 hour, that continued after 4 and 8 hours post treatment. GLP-1 peptide treated mice also manifested considerable blood glucose reduction after 1 hour, however in contrast their blood glucose levels did not continue to decline at the rate of Surrobody treated mice. In fact their blood glucose levels at 4 and 8 hours post treatment started to approach levels of control Surrobody (Surrobody lacking peptide fusion) treated mice, indicating substantial loss of GLP-1 in these synthetic peptide treated group.

### Example 5: Reduction of plasma glucose following glucose tolerance testing in diabetic mice treated with GLP-1 conjugated null Surrobodies

GLP-1 conjugated null Surrobodies can be evaluated for its effects to improve glucose tolerance in db/db mice. We will use db/db diabetic mice to examine the insulinotropic effects of GLP-1 (Ser8) Surrobodies to lowering both basal and peak blood glucose levels 1, 24, & 48 hours after intravenous administration. Specifically, blood glucose levels will be measured 1 hour prior to and 1 hour after intravenous administration of 1 mg/kg GLP-1 (Ser8) Surrobody. Following the blood draw 1 hour after Surrobody administration mice will be injected intraperitoneally with 2g/kg glucose and their blood glucose levels monitored at 15, 30, 60, 90, and 120 minutes after glucose administration. We will test two additional groups of mice in similar fashions, except the glucose tolerance tests will occur at 24 and 48 hours following GLP-1 (Ser8) Surrobody administration.

### Example 6: Peptide fused functionally null Surrobody stability in vivo

Recombinant Exendin-4 Surrobodies were tested to determine the stability of the recombinant peptide fusion to the functionally null Surrobody. To test the durability of the peptide fusion, we recombinantly conjugated an Exendin-4 peptide (Met14Leu) to the amino-terminus of a surrogate light chain fusion (SEQ ID NO: 38 - Exendin-4 Fusion 1). The Surrobody was administered via intravenous injection. A small tail bleed was performed 1 hour after injection to establish the maximal (100% level) serum concentrations in the mice. At 24 hours the subjects were sacrificed and terminal cardiac bleeds were used to prepare serum for analysis. Serum levels of the functionally null Surrobody was examined by quantitative sandwich ELISA that utilized an anti-surrogate light chain capture and detection with antihuman Fc detection antibodies, while levels of Exendin-4 peptide stability on the functionally null Surrobodies were examined by quantitative sandwich ELISA utilizing an anti- surrogate light chain and detection with anti-Exendin polyclonal detection antibodies. The table below shows the % remaining after 24 hours.

| | Exendin-4 peptide fusion | Surrobody |
|---|---|---|
| Exendin-4 SgG | 36% | 43% |

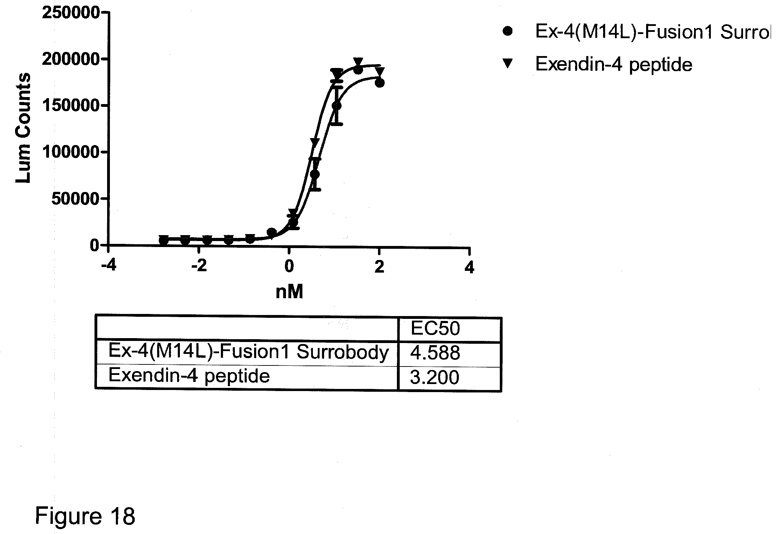

Figure 18 demonstrates that an Exendin-4 Surrobody Maintain Full Potency and Efficacy in vitro.

The sequence of the Exendin-4 peptide with and without the methionie to leucine substitution are provided below.
HGEGTFTSDLSKQMEEEAVRLFIEWLKNGGPSSGAPPPS - SEQ ID NO:35
HGEGTFTSDLSKQ**L**EEEAVRLFIEWLKNGGPSSGAPPPS - SEQ ID NO:36.
Other Exendin-4 constructs may be tested (see Figure 1).

## Claims

1. A fusion molecule comprising a first polypeptide and a second polypeptide, wherein said second polypeptide is a Surrobody™ or Surrobody™ fragment comprising one or more functionally null binding regions, wherein a functionally null binding region does not bind to any self-target or foreign target present in a recipient living organism, fused to and capable of extending the *in vivo* half-life of said first polypeptide.

2. The fusion molecule of claim 1 wherein the first polypeptide is biologically active or not biologically active, wherein the fusion molecule optionally further comprises a biologically active molecule conjugated to said polypeptide.

3. The fusion molecule of claim 1 wherein the Surrobody™ comprises (i) a VpreB and/or a λ5 sequence; or (ii) a Vκ-like and/or a JCκ-like sequence.

4. The fusion molecule of claim 2 wherein the biologically active peptide or polypeptide is
(i) fused to a functionally null surrogate light chain;
(ii) fused to the C-terminus of the functionally null surrogate light chain;
(iii) fused to the N-terminus of the functionally null surrogate light chain; or
(iv) inserted into the functionally null surrogate light chain.

5. The fusion molecule of claim 4 wherein said surrogate light chain comprises VpreB and λ5 sequences non-covalently associated with each other.

6. The fusion molecule of claim 5 wherein the biologically active peptide or polypeptide is fused to both the N- terminus and the C-terminus of at least one of the VpreB and λ5 sequences.

7. The fusion molecule of any one of claims 1 to 6, wherein the first polypeptide is selected from the group consisting of interferon alpha (IFN-α), interferon beta (IFN-β), calcitonin, parathyroid hormone, BAFF-r, TALI, FSH, Interleukin-2, erythropoietin (Epo), thrombopoietin (Tpo) G-CSF, GM-CSF, Factor VII, DNAse, hirudin, urokinase, streptokinasae, Growth hormone, Glucagon, Kremen-1, Kremen-2, HGF, FGF-21, GLP-1, Exendin-4, Oxyntomodulin, amylin, PACAP, T20 HIV inhibitory peptide, IL-22, Thrombospondin peptide fragments, BMP-7, CTLA-IV, t-PA, Flt-1, IL-lra, Insulin, melanocortin, herstatin, amylin, conotoxins, TNF-RI, GITR, Gastrin, Epo agonist peptide mimetics, Tpo agonist peptide mimetics, antibody fragments, single-chain antibodies (scFv), nobodies, avimers, phylomers, DARPins, anti-calMs, adnectins, and tetranectins.

## Patentansprüche

1. Fusionsmolekül, umfassend ein erstes Polypeptid und ein zweites Polypeptid, wobei das zweite Polypeptid ein Surrobody™ oder ein Surrobody™-Fragment ist, der/das eine oder mehrere funktional nicht bindende Regionen umfasst, wobei eine funktional nicht bindende Region an kein körpereigenes oder fremdes, in einem Empfängerlebewesen vorhandenes Target bindet, und das mit dem ersten Polypeptid fusioniert ist und dazu fähig ist, dessen Halbwertszeit in vivo zu verlängern.

2. Fusionsmolekül nach Anspruch 1, wobei das erste Polypeptid biologisch aktiv oder nicht biologisch aktiv ist, wobei das Fusionsmolekül gegebenenfalls weiters ein biologisch aktives Molekül umfasst, das an das Polypeptid konjugiert ist.

3. Fusionsmolekül nach Anspruch 1, wobei der Surrobody™ (i) eine VpreB- und/oder eine λ5-Sequenz oder (ii) eine Vκ-ähnliche und/oder eine JCκ-ähnliche Sequenz umfasst.

4. Fusionsmolekül nach Anspruch 2, wobei das biologisch aktive Peptid oder Polypeptid
(i) mit einer funktionslosen Surrogat-Leichtkette fusioniert ist;
(ii) mit dem C-Terminus der funktionslosen Surrogat-Leichtkette fusioniert ist;
(iii) mit dem N-Terminus der funktionslosen Surrogat-Leichtkette fusioniert ist; oder
(iv) in die funktionslose Surrogat-Leichtkette insertiert ist.

5. Fusionsmolekül nach Anspruch 4, wobei die Surrogat-Leichtkette VpreB- und λ5-Sequenzen umfasst, die nicht-kovalent aneinander gebunden sind.

6. Fusionsmolekül nach Anspruch 5, wobei das biologisch aktive Peptid oder Polypeptid sowohl mit dem N-Terminus als auch mit dem C-Terminus von zumindest einer aus den VpreB- und λ5-Sequenzen fusioniert ist.

7. Fusionsmolekül nach einem der Ansprüche 1 bis 6, wobei das erste Polypeptid aus der Gruppe bestehend aus Interferon-alpha (IFN-α), Interferon-beta (IFN-β), Calcitonin, Nebenschilddrüsenhormon, BAFF-r, TALI, FSH, Interleukin-2, Erythropoietin (Epo), Thrombopoietin (Tpo), G-CSF, GM-CSF, Faktor VII, DNAse, Hirudin, Urokinase, Streptokinase, Wachstumshormon, Glucagon, Kremen-1, Kremen-2, HGF, FGF-21, GLP-1, Exendin-4, Oxyntomodulin, Amylin, PACAP, T20 HIV-Inhibitorpeptid, IL-22, Thrombospondinpeptidfragmenten, BMP-7, CTLA-IV, t-PA, Flt-1, IL-1ra, Insulin, Melanocortin, Herstatin, Amylin, Conotoxinen, TNF-RI, GITR, Gastrin, Epo-Agonist-Peptidmimetika, Tpo-Agonist-Peptidmimetika, Antikörperfragmenten, einkettigen Antikörpern (scFv), Nullkörpern, Avimeren, Phylomeren, DARPinen, Anti-calM-Antikörpern, Adnektinen und Tetranektinen ausgewählt ist.

## Revendications

1. Molécule de fusion comprenant un premier polypeptide et un second polypeptide, dans laquelle ledit second polypeptide est un Surrobody™ ou un fragment de Surrobody™ comprenant une ou plusieurs régions de liaison fonctionnellement nulles, où une région de liaison fonctionnellement nulle ne se lie pas à une quelconque auto-cible ou cible étrangère présente chez un organisme vivant receveur, fusionné à et capable de prolonger la demi-vie *in vivo* dudit premier polypeptide.

2. Molécule de fusion selon la revendication 1, dans laquelle le premier polypeptide est biologiquement actif ou non biologiquement actif, où la molécule de fusion comprend facultativement en outre une molécule biologiquement active conjuguée audit polypeptide.

3. Molécule de fusion selon la revendication 1, dans laquelle le Surrobody™ comprend (i) une séquence VpreB et/ou λ5 ; ou (ii) une séquence de type Vκ et/ou de type JCκ.

4. Molécule de fusion selon la revendication 2, dans laquelle le peptide ou polypeptide biologiquement actif est
(i) fusionné à une chaîne légère de substitut fonctionnellement nulle ;
(ii) fusionné à l'extrémité C-terminale de la chaîne légère de substitut fonctionnellement nulle ;
(iii) fusionné à l'extrémité N-terminale de la chaîne légère de substitut fonctionnellement nulle ; ou
(iv) inséré dans la chaîne légère de substitut fonctionnellement nulle.

5. Molécule de fusion selon la revendication 4, dans laquelle ladite chaîne légère de substitut comprend des séquences VpreB et λ5 associées l'une à l'autre de façon non covalente.

6. Molécule de fusion selon la revendication 5, dans laquelle le peptide ou polypeptide biologiquement actif est fusionné à la fois à l'extrémité N-terminale et à l'extrémité C-terminale d'au moins une des séquences VpreB et λ5.

7. Molécule de fusion selon l'une quelconque des revendications 1 à 6, dans laquelle le premier polypeptide est sélectionné dans le groupe consistant en l'interféron alpha (IFN-α), l'interféron bêta (IFN-β), la calcitonine, l'hormone parathyroïdienne, BAFF-r, TALI, la FSH, l'interleukine-2, l'érythropoïétine (Epo), la thrombopoïétine (Tpo), le G-CSF, le GM-CSF, le facteur VII, la DNAse, l'hirudine, l'urokinase, la streptokinase, l'hormone de croissance, le glucagon, Kremen-1, Kremen-2, le HGF, le FGF-21, le GLP-1, l'exendine-4, l'oxyntomoduline, l'amyline, PACAP, le peptide T20 inhibiteur du VIH, l'IL-22, des fragments peptidiques de la thrombospondine, la BMP-7, CTLA-IV, le t-PA, Flt-1, l'IL-1ra, l'insuline, la mélanocortine, l'herstatine, l'amyline, des conotoxines, le TNF-RI, GITR, la gastrine, des peptidomimétiques agonistes de l'Epo, des peptidomimétiques agonistes de la Tpo, des fragments d'anticorps, des anticorps à chaîne unique (scFv), des « nobodies », des avimères, des phylomères, des DARPines, des anti-calM, des adnectines, et des tétranectines.
